# EUROPEAN PATENT APPLICATION

(11) **EP 1 935 992 A2**
(43) Date of publication of application: **25.06.2008**
(21) Application number: 08101207.2
(22) Date of filing: 22.12.2004
(51) Int. Cl.: C12Q 1/70

(54) **Multiplexed nucleic acid analysis with high specificity for human papilloma virus**

(30) Priority: 23.12.2003 US 532681 P; 26.03.2004 US 556737 P
(62) Divisional of application: 04815560.0
(71) Applicant: Autogenomics, Inc., Carlsbad, CA 92009 (US)
(72) Inventor: Ke, Song-Hua, San Diego, CA 92130 (US); Hudspeth, Richard Loren, San Diego, CA 92131 (US); Mahant, Vijay K., Murrieta, CA 92562 (US)
(74) Representative: Rupp, Christian

(57) **Abstract**

The invention concerns human papillomavirus (HPV) recognition sequences for use in the detection of HPV. The recognition sequences may be used in test kits and methods employing amplification and extension primers that are selected to allow multiplex PCR and extension at increased specificity. Preferably, the extension primers include a tag that hybridizes with a capture probe on a biochip, wherein the tag is distinct from the target nucleic acid sequence to be analyzed. Further preferred kits include a biochip and various instructions.

## Description

This application claims the benefit of our U.S. Provisional Patent Applications with the serial numbers 60/532,681 (filed December 23, 2003) and 60/556,737 (filed March 26, 2004), both of which are incorporated by reference herein.

### Field of The Invention

The field of the invention is genetic diagnostics, and especially as it relates to multiplex analysis of a single sample.

### Background of The Invention

Despite recent advances in molecular diagnostics, numerous difficulties still remain. Among other problems, analysis of multiple potential genetic changes in a sample suspected to include a virus or oncogene frequently lead to false positive results, or fail to identify all potential changes as the number of such changes increases. Similar difficulties arise where one or more organisms are subject to genotyping or other genetic analysis.

For example, human papillomavirus (HPV) is now considered a major cause of cervical cancer, killing more than 200,000 women around the world each year. The HPV virus is relatively common and more than 100 distinct types of HPV have been identified, some of which are considered "high-risk" for the development of cancer. Detection of such high-risk types of HPV has significant impact on diagnosis, prevention, treatment and management of cervical cancer in HPV-infected women.

To date, most molecular methods for HPV detection and typing rely on hybridization technologies, including southern blot, dot blot, line blot, *and in situ* hybridization. For example, HybridCapture II from Digene is a nucleic acid hybridization microplate assay based on chemiluminescence for the qualitative detection, and differentiating low-risk from high-risk groups. Other commercially available tests employ similar methods and may detect the presence of various types of HPV in a patient sample. However, known HPV typing methods based on hybridization often lack specificity due to cross-hybridization. Cross-hybridization may result in a false positive signal due to closely related types of HPV (e.g., where a target DNA has only a single or few mismatches to the probes being used). Thus, the accuracy of the test results may be compromised with samples containing multiple viral types with closely related sequences.

To overcome problems associated with cross-hybridization, a number of approaches have been taken. Typically, most of the improvements focus on exact control of the stringency conditions. For example, the specificity of hybridization can be controlled by temperature. However, temperature-specific hybridization may lead to false positive results if probes have a high degree of sequence similarity.

Other efforts included the use of peptide nucleic acids (PNA), a universal base stretch, or modified bases (e.g., super G and C) to alter or otherwise affect hybridization/melting temperature of duplexes. Still further known methods involve use of conformationally locked DNA *(e.g.,* to increase duplex stability), etc. While most of such approaches have provided at least some advantages, various problems nevertheless remain. Among other things, currently known approaches tend to fail to provide a significant difference between the melting and/or hybridization temperature of a perfectly matched hybrid and a single base mismatched hybrid.

Therefore, while numerous methods for nucleic acid based testing of HPV and other pathogens are known in the art, all or almost all of them suffer from various problems, which are even more aggravated, where such analysis is performed in a multiplex environment (e.g., a biochip). Consequently, there is still a need to provide improved methods and compositions for molecular diagnostics.

### Summary of the Invention

The present invention is directed to compositions and methods for genetic diagnostics in which specificity is substantially improved by using a combination of selected multiplex amplification primers and selected multiplex extension primers, wherein the sequences of the primers are designed to maximize hybridization specificity and extension selectivity in a multiplex reaction.

In one aspect of the inventive subject matter, a multiplex diagnostic kit includes a plurality of amplification primer pairs, and a plurality of extension primers, wherein each of the plurality of amplification primer pairs has a sequence such that (a) a plurality of amplicons produced from a target nucleic acid using the plurality of amplification primer pairs, respectively, includes a sequence difference (mutated position) in a target nucleic acid, (b) the plurality of amplicons is produced in a PCR reaction using the same amplification profile, and wherein each of the plurality of extension primers has a sequence such that (c) each of the plurality of extension primers specifically hybridizes to each of the plurality of amplicons at the same extension temperature, respectively, and selective primer extension for each of the plurality of extension primers is achieved at the same extension profile. Amplification primers and extension primers are most preferably those described in SEQ ID Ax, SEQ ID Bx, and SEQ ID Cx, with x being an integer between 1 and 24.

Particularly preferred kits further include a biochip to which are coupled in a plurality of distinct positions a plurality of distinct capture probes, respectively, and wherein each of the plurality of capture probes hybridizes with a portion of each of the extension primers, respectively. Most preferably, each of the plurality of the distinct capture probes has a unique sequence distinct from the target nucleic acid. Additionally, contemplated kits can include DNA-dependent DNA polymerase (e.g. thermostable, or specifically modified and/or isolated for primer extension), and/or an instruction (e.g., to perform the PCR reaction and primer extension in a single tube).

In another aspect of the inventive subject matter, a multiplex diagnostic kit includes at least two forward amplification primers having a sequence according to SEQ ID Ax and Ay, at least two backward amplification primers having a sequence according to SEQ ID Bx and By, and at least two extension primers having a sequence according to SEQ ID Cx and Cy, wherein x and y are integers between 1 and 24 and not the same. Such kits can further include an instruction to perform a multiplex PCR using the at least two forward amplification primers and the at least two backward amplification primers using the same amplification profile, and optionally an instruction to perform a primer extension reaction using the at least two extension primers at the same extension profile (typically in a single test tube).

Additionally, or alternatively, a biochip is included in the test kit to which are coupled in a plurality of distinct positions a plurality of distinct capture probes, respectively, and wherein each of the plurality of capture probes hybridizes with a portion of each of the extension primers, respectively. Most preferably, each of the plurality of the distinct capture probes has a unique sequence distinct from a target nucleic acid to which the amplification primers bind. Where desired, contemplated kits may also include a reagent and/or an enzyme.

In a still further contemplated aspect of the inventive subject matter, a synthetic nucleic acid has less than sixty (most preferably less than forty) nucleotides and comprises an HPV recognition sequence selected from the group consisting of SEQ ID Ax, SEQ ID Bx, and SEQ ID Cx, wherein X is an integer between 1 and 24, wherein no more than two nucleotides in the HPV recognition sequence are replaced by N (A, G, C, or T). Such synthetic nucleic acids especially include those having SEQ ID Cx and further comprise a plurality of nucleotides at the 5'-terminus that have less than 60% homology to a target sequence to which the nucleic acid hybridizes.

Various objects, features, aspects and advantages of the present invention will become more apparent from the following detailed description of preferred embodiments of the invention.

### Detailed Description

The inventors have unexpectedly discovered that a plurality of potential variants of a single gene can be identified in a single sample using a multiplex test in which amplification primers are used to specifically amplify a target sequence in that gene, wherein the amplicon includes at least one of the potential variants, and wherein extension primers are used to form an extension product that is specific to a variant of the gene.

It should be especially noted that the specificity in such tests is substantially increased over conventional methods by the manner of primer selection. Specifically, the amplification primers are selected to have a sequence such that (a) a plurality of amplicons produced from a target nucleic acid using the amplification primers include sequence difference in a target nucleic acid, and (b) the plurality of amplicons is produced in a PCR reaction using the same amplification profile. In the same test, the extension primers are selected to have a sequence such that (c) the extension primers specifically hybridize to the corresponding amplicons at the same extension temperature (preferably such that the 3'-end of each of the extension primers corresponds to a complementary position of the mutated position), and (d) selective primer extension for each of the extension primers is achieved at the same extension temperature.

Most preferably, the 5'-end of the extension primers further includes a tag (zipcode sequence) that is substantially not (typically less than 70%, and most typically less than 50%) complementary to the sequence of the amplicons and/or the sequence of the target gene, wherein the zipcode sequence is employed to hybridize with a capture probe (preferably on a biochip in a predetermined position). While not limiting to the inventive subject matter, the zipcode has typically a length between about two and twenty, more preferably between five and fifteen, and most preferably between eight and twelve nucleotides, wherein the tags of each of the extension primers are distinct (*i.e.*, have a unique sequence), and wherein the zipcodes (and with that the distinct capture probes) have a unique sequence distinct from the target nucleic acid.

With respect to the particular sequences of the amplification primers and the extension primers, it should be recognized that all sequences are deemed suitable and that the specific sequences will predominantly depend on the particular nature of the target nucleic acid and type of sequence difference that is to be detected. For example, suitable target nucleic acids include native and recombinant DNA *(e.g.,* linear, circular, etc.), RNA *(e.g.,* snRNA, hnRNA, mRNA, etc.), synthetic nucleic acids (e.g., phosphorothioates, PNA, etc.), all of which may be present in, or isolated from a biological source (e.g., biopsy, cell culture, swab, filtrate, plant material, etc.), a non-biological source (e.g., food, soil, water, oil, etc.), or may be entirely synthetic (e.g., on solid phase). Thus, it should be recognized that the length of contemplated target nucleic acids may vary considerably, and is typically between about 50 nucleotides to the length of an entire genome, chromosome, vector, chromosomal fragment, or transcript. Most preferably, the target nucleic acid is a viral or bacterial genome, or a nucleic acid comprising an oncogene, tumor suppressor gene, or other gene that is associated with a predisposition or presence of a disease. In the example, below, a particularly preferred target DNA is a viral DNA, and especially HPV DNA.

It is generally preferred that the amplification primers have a length of between about 12 to 50 nucleotides, and more preferably between about 16 to 30 nucleotides, wherein the amplification primers may additionally (or optionally) include one or more nucleotides that provide one or more desirable properties. For example, contemplated amplification primers may include one or more nucleotides that render the primer (and/or amplicon) quantifiable and typical examples include radiolabeled nucleotides, fluorescence-labeled nucleotides, etc. In another example, contemplated amplification primers may also include one or more nucleotides that will facilitate specific isolation of the primer and/or amplicon (e.g., biotinylated nucleotide). Thus, amplicons generated by contemplated methods may be quantified to normalize a test result, especially where the test result provides a quantitative measure. Amplicons generated by contemplated tests will typically have a length of between about 50 to several thousand nucleotides.

Similarly, preferred extension primers can have a length of between about 12 to 50 nucleotides, and more preferably between about 16 to 30 nucleotides, wherein the extension primers may additionally (or optionally) include one or more nucleotides that provide for one or more desirable properties. For example, particularly contemplated extension primers can include several additional nucleotides that allow specific hybridization of the additional nucleotides to a capture probe. Most preferably, the additional nucleotides have a sequence that is distinct from the sequence of the target nucleic acid (and even more typically of the amplicon). Therefore, capture of the extension product is independent of the target sequence, which further increases selectivity of the test. For example, various SNP-specific tests known in the art use solid-phase or otherwise immobilized extension primers, which tend to produce false positive results where the sequence difference among various mutant sequences allows cross-hybridization. Of course, it should be recognized that the sequence of the extension primer is selected from a sequence available in the amplicon.

Depending on the particular target nucleic acid, contemplated amplification and/or extension primers can also include modified nucleotides and/or have one or more ambiguous positions (*i.e.*, a position in which different nucleotides are present among otherwise identical primers). Ambiguous positions are denoted using the IUPAC nomenclature (R is A or G, Y is C or T, S is C or G, W is A or T, M is G or T, M is A or C, B is C or G or T, D is A or G or T, H is A or C or T, V is A or C or G, and N is A or C or G or T). Therefore, contemplated amplification and/or extension primers may have a single defined Tₘ at a particular solvent and temperature, or several distinct Tₘ.

However, it should be recognized that the amplification primers are chosen such that a multiplex PCR using the amplification primers can be performed using a single amplification profile (wherein the term "amplification profile" refers to a specific combination of denature temperature and time, anneal temperature and time, and polymerization temperature and time), and that all amplicons produced from the multiplex PCR can be used for a primer extension reaction using the extension primers at a single extension temperature (wherein the term "extension temperature" refers to a specific combination of hybridization temperature and time and polymerization temperature and time). Preferred extension products are typically in the range of about 50 to several thousand bases, and it is especially preferred that the extension product includes one or more detectable (and more preferably quantifiable) label. For example, the extension reaction may be performed using one or more directly or indirectly labeled nucleotides, including nucleotides that carry a fluorescent, luminescent, or radioactive label (wherein the molar fraction of labeled nucleotide may be adjusted as appropriate), and/or nucleotides that carry an affinity marker (e.g., biotin, digitoxin) that binds a labeled compound or compound that can otherwise be detected and/or quantified.

It is generally preferred that the extension primer has a sequence and is positioned such that proper hybridization of the extension primer (and especially correct hybridization of the terminal three 3' bases, more preferably terminal two 3' bases, and most preferably terminal 3' base) with the target nucleic acid will result in a detectable extension event. Typically the detectable event is a DNA polymerase-dependent DNA synthesis, wherein at least one of the nucleotides is labeled. Alternatively, the detectable event may also be a DNA ligation using a labeled fragment that abuts with it's 5'-end the 3'-end of the extension primer. With respect to the type of sequence difference that can be detected using contemplated methods, it should be recognized that all known sequence differences are suitable so long as information is available that allows design of the amplification primers and the extension primers. Thus, contemplated differences include deletions, insertions, translocations, and substitutions (*e.g*., transversion or transition). Furthermore, it should be noted that contemplated sequence differences also include sequence differences found in distinct viral genotypes. Thus, the nucleotide differences between or among various genotypes of a viral species are also considered mutations herein.

Detection is preferably carried out on a biochip or other carrier onto which are immobilized in predetermined positions a plurality of capture probes that hybridize with at least a portion of the extension primer and/or extension product. Therefore, contemplated diagnostic kits can also include (next to contemplated amplification primers and/or extension primers) a biochip to which are coupled in a plurality of distinct positions a plurality of distinct capture probes, respectively, and wherein each of the plurality of capture probes hybridizes with at least a portion of each of the extension primers, respectively. In still further preferred aspects, contemplated capture probes may also include a fluorescent label, wherein the emission of the label is most preferably at a wavelength different from the detection wavelength of the extension product (e.g., Cy5 for the capture probe and Cy3 for the extension product). Among other advantages, such configurations allow normalization and/or calibration of a signal from the extension product. Additionally, or alternatively, suitable kits may include various enzymes (e.g., DNA-dependent DNA polymerase, ligase, etc.), buffers, and other reagents (e.g., labeled and unlabeled nucleotides).

**Table 1A** depicts exemplary forward and backward amplification primers and corresponding extension primers for detection of genetic variants of HPV, wherein an extension primer in the same row as a forward and backward amplification primer will bind to the amplicon produced by the amplification primers. It should be noted that the primers in the Table 1A may include degenerate nucleotide positions. Therefore, primers with degenerate positions represent both individual sequences as well as mixtures of sequences defined by the ambiguity codes (e.g., ASA may represent AGA individually or ACA individually, but also a mixture of ACA and AGA together). **Table 1B** depicts an exemplary selection of certain primers of Table 1A with non-degenerate sequences.

The amplification primers and extension primers correspond to the sequences provided in the sequence listing below, wherein SEQ ID: A1-A24 of Table 1A correspond to Sequence Numbers 1-24 of the sequence listing, respectively, wherein SEQ ID: B1-B24 of Table 1 correspond to Sequence Numbers 25-48 of the sequence listing, respectively, and wherein SEQ ID: C1-C24 of Table 1 correspond to Sequence Numbers 49-72 of the sequence listing, respectively. Furthermore, SEQ ID: A25-A26 of Table 1B correspond to Sequence Numbers 73-74 of the sequence listing, respectively, and SEQ ID: B25-B41 of Table 1B correspond to Sequence Numbers 75-91 of the sequence listing, respectively.

The primers according to Tables 1A and 1B (and other primers contemplated above) can further be modified to yield a synthetic nucleic acid having less than seventy, more preferably less than sixty nucleotides, and most preferably less than 50 nucleotides and comprising an HPV recognition sequence selected from the group consisting of SEQ ID Ax, SEQ ID Bx, and SEQ ID Cx, wherein X is an integer between 1 and 24, wherein no more than three, and most preferably no more than two nucleotides in the HPV recognition sequence are replaced by N (as defined in IUPAC nomenclature) or other non-natural nucleotide. Furthermore, nucleotides according to SEQ ID Cx may further include a plurality of nucleotides at the 5'-terminus that have less than 70%, more typically less than 60%, and most typically less than 40% homology to a target sequence to which the nucleic acid hybridizes.

Especially contemplated kits will include at least two, more typically at least three to five, and most typically at least ten to twenty of the amplification primer pairs, and/or corresponding extension primers. In such kits, the PCR reaction and/or the primer extension is preferably performed in a single tube, which may be reflected in an instruction accompanying such kits. Alternatively, at least one of the PCR reaction and the primer extension can also be performed in an automated analyzer. Contemplated instructions may further provide information to perform the multiplex PCR using at least two forward amplification primers and at least two backward amplification primers using the same amplification profile, and/or information to perform the primer extension reaction using at least two extension primers at the same extension temperature.

While not wishing to be bound by a particular hypothesis or theory, the inventors contemplate that the specificity of the tests according to the inventive subject matter is further improved by virtue of the fact that at least one of the hybridizations, and more preferably both hybridizations *(i.e.,* for amplification and extension) is performed in solution rather than on a solid phase (which is thought to interfere with hybridization specificity). Furthermore, by the particular choice of primer selection, and especially by targeting distinguishing sequences among a plurality of otherwise similar or identical sequences, hybridization specificity of the amplification and/or extension primers is further increased.

### Experiments

The following experiments were performed to provide exemplary guidance for a test to detect and genotype an HPV virus from a human sample. Here, HPV DNA was isolated from a pap smear using the Qiagen DNA isolation kit and an aliquot of the eluent was subjected to an off-line multiplex PCR using the forward and backward amplification primers with the SEQ ID A1-A24 and B1-B24, respectively. The PCR conditions were as follows:

### HPV Multiplex PCR

To 1 uL sample were added 18.75 uL HPV Amplification Solution, 0.25 uL (1.25 units) Platinum Taq Polymerase (Invitrogen) to a final volume of 20 uL. The HPV Amplification Solution was 21.34 mM Tris-HCL (pH 8.4), 53.35 mM KCL, 2.67 mM MgCl2, 33.34 uM dATP, 33.34 uM dGTP, 33.34 uM dTTP, 6.67 uM dCTP, and 26.68 to 80.03 nM for each of the forward and backward amplification primers.

A 24-plex PCR using the primers of Table 1A was performed using the following amplification profile: Activation of Platinum Taq polymerase was performed by incubation at 94°C for 1 min followed by 40 cycles of 5 sec denaturation at 94°C, 30 sec annealing at 52°C and 40 sec elongation at 72°C. The contents of the multiplex PCR was then used in a subsequent primer extension using the extension oligos with the SEQ ID C1-C24 in a single well of a multi-well plate that was disposed in an automated analyzer as follows:

### HPV Primer Extension

The primer extension was performed in a 24 well plate in an automated analyzer using temperature controlled incubation of the extension mixture and reagents as follows:

To the 20 uL volume from the multiplex PCR reaction were added 20 uL HPV Primer Extension Solution to a final volume of 40 uL. The HPV Primer Extension Solution was 20 mM Tris-HCL (pH 8.4), 50 mM KCL, 2.5 mM MgCl2, 31.25 uM dATP, 31.25 uM dGTP, 31.25 uM dTTP, 5 uM cy5dCTP, and 25 nM for each of the extension primers SEQ ID C1-C24. The extension profile was as follows: The PCR reaction was denatured at 94°C for 1 min followed by 40 cycles of 5 sec at 94°C and 10 sec at 51°C.

### HPV Detection and Genotyping

The entire volume of the extension reaction was then transferred onto a biochip that included in predetermined position a plurality of capture nucleotides as described in our copending International applications WO 03/050591 and WO 02/057416, both of which are incorporated by reference herein. Extension products were detected by fluorescence detection using the Cy label on the extension product (which is only formed where the extension primer forms a perfect hybrid with the target nucleic acid), wherein a predetermined position of the capture primer corresponds to a predetermined HPV genotype. Genotyping and detection was confirmed using the reference test HC2 HPV DNA test from Digene Corporation. The results from the test according to the inventive subject matter and the commercially available test correlated 100% as shown in Table 2 in which HR represents "high-risk" genotype (with particular genotype provided in parentheses), LR represents "low-risk" genotype (with particular genotype provided in parentheses), and neg represents negative result.

**Table 2**

| **INVENTIVE TEST (24 PLEX)** | **REFERENCE TEST** |
|---|---|
| HR (45) | HR |
| neg | neg |
| neg | neg |
| HR (31, 66) | HR, LR |
| HR (16, 26, 33, 52, 82), LR (6) | HR, LR |
| HR (16) | HR, LR |
| HR (35, 58, 66) | HR, LR |
| HR (18, 53, 56), LR (42) | HR, LR |
| HR (39, 58) | HR |
| HR (35) | HR |
| neg | neg |
| HR (35, 52) | HR |
| HR (18) | HR |
| HR(18, 35) | HR, LR |

Thus, specific embodiments and applications of multiplexed HPV nucleic acid analysis with improved specificity have been disclosed. It should be apparent, however, to those skilled in the art that many more modifications besides those already described are possible without departing from the inventive concepts herein. The inventive subject matter, therefore, is not to be restricted except in the spirit of the appended claims. Moreover, in interpreting both the specification and the claims, all terms should be interpreted in the broadest possible manner consistent with the context. In particular, the terms "comprises" and "comprising" should be interpreted as referring to elements, components, or steps in a non-exclusive manner, indicating that the referenced elements, components, or steps may be present, or utilized, or combined with other elements, components, or steps that are not expressly referenced. Furthermore, where a definition or use of a term in a reference, which is incorporated by reference herein is inconsistent or contrary to the definition of that term provided herein, the definition of that term provided herein applies and the definition of that term in the reference does not apply.

SEQUENCE LISTING

<110> Autogenomics

<120> MULTIPLEXED HPV NUCLEIC ACID ANALYSIS WITH IMPROVED SPECIFICITY

<130> 100788.0023PCT

<150> US 60/532681
<151> 2003-12-23

<150> US 60/556737
<151> 2004-03-26

<160> 91

<170> PatentIn version 3.2

<210> 1
<211> 23
<212> DNA
<213> Artificial

<220>
<223> Artificial sequence; IUPAC ambiguity code applies for all
nucleotides other than A, C, G, and T.

<220>
<221> misc_feature
<222> (1)..(23)
<223> Forward Primer for HPV Type 6

<400> 1
gcaacaacag ttgaagaaga aac 23

<210> 2
<211> 23
<212> DNA
<213> Artificial

<220>
<223> Artificial sequence; IUPAC ambiguity code applies for all
nucleotides other than A, C, G, and T.

<220>
<221> misc_feature
<222> (1)..(23)
<223> Forward Primer for HPV Type 11

<400> 2
gcacctacag tagaagaaga aac 23

<210> 3
<211> 20
<212> DNA

<213> Artificial

<220>
<223> Artificial sequence

<220>
<221> misc_feature
<222> (1)..(20)
<223> Forward Primer for HPV Type 16

<220>
<221> misc_feature
<222> (12)..(12)
<223> K is G or T

<220>
<221> misc_feature
<222> (15)..(15)
<223> R is A or G

<400> 3
gtatatagag akggraatcc 20

<210> 4
<211> 21
<212> DNA
<213> Artificial

<220>
<223> Artificial sequence

<220>
<221> misc_feature
<222> (1)..(21)
<223> Forward Primer for HPV Type 18

<400> 4
gtgtatagag acagtatacc g 21

<210> 5
<211> 20
<212> DNA
<213> Artificial

<220>
<223> Artificial sequence

<220>
<221> misc_feature
<222> (1)..(20)
<223> Forward Primer for HPV Type 26

<400> 5
gtatatagag ataggagtcc 20

<210> 6
<211> 20
<212> DNA
<213> Artificial

<220>
<223> Artificial sequence


<220>
<221> misc_feature
<222> (1)..(20)
<223> Forward Primer for HPV Type 31

<400> 6
gtatataggg acgacacacc                                20


<210> 7
<211> 20
<212> DNA
<213> Artificial

<220>
<223> Artificial sequence


<220>
<221> misc_feature
<222> (1)..(20)
<223> Forward Primer for HPV Type 33

<220>
<221> misc_feature
<222> (12)..(12)
<223> K is G or T

<220>
<221> misc_feature
<222> (15)..(15)
<223> R is A or G

<400> 7
gtatatagag akggraatcc                                20


<210> 8
<211> 20
<212> DNA
<213> Artificial

<220>
<223> Artificial sequence


<220>
<221> misc_feature

<222> (1)..(20)
<223> Forward Primer for HPV Type 35

<400> 8
gtatatagag aaggccagcc                                20

<210> 9
<211> 20
<212> DNA
<213> Artificial

<220>
<223> Artificial sequence

<220>
<221> misc_feature
<222> (1)..(20)
<223> Forward Primer for HPV Type 39

<400> 9
gtatataggg acggggaacc                                20

<210> 10
<211> 23
<212> DNA
<213> Artificial

<220>
<223> Artificial sequence

<220>
<221> misc_feature
<222> (1)..(23)
<223> Forward Primer for HPV Type 42

<400> 10
tggtatacag tggagaaaga aac                            23

<210> 11
<211> 23
<212> DNA
<213> Artificial

<220>
<223> Artificial sequence

<220>
<221> misc_feature
<222> (1)..(23)
<223> Forward Primer for HPV Type 43

<400> 11
gcagatactg tagaagaaga aac                            23

<210> 12
<211> 23
<212> DNA
<213> Artificial

<220>
<223> Artificial sequence


<220>
<221> misc_feature
<222> (1)..(23)
<223> Forward Primer for HPV Type 44

<400> 12
gcagtaacag tggaagaaga aac                    23


<210> 13
<211> 20
<212> DNA
<213> Artificial

<220>
<223> Artificial sequence


<220>
<221> misc_feature
<222> (1)..(20)
<223> Forward Primer for HPV Type 45

<400> 13
gtgtatagag actgtatagc                    20


<210> 14
<211> 26
<212> DNA
<213> Artificial

<220>
<223> Artificial sequence


<220>
<221> misc_feature
<222> (1)..(26)
<223> Forward Primer for HPV Type 51

<400> 14
gattgtatat agggataata atccat                    26


<210> 15
<211> 25
<212> DNA

<213> Artificial

<220>
<223> Artificial sequence

<220>
<221> misc_feature
<222> (1)..(25)
<223> Forward Primer for HPV Type 52

<400> 15
gaatagtata tagagacaat aatcc 25

<210> 16
<211> 20
<212> DNA
<213> Artificial

<220>
<223> Artificial sequence

<220>
<221> misc_feature
<222> (1)..(20)
<223> Forward Primer for HPV Type 53

<400> 16
gtgtatagag acgggtatcc 20

<210> 17
<211> 20
<212> DNA
<213> Artificial

<220>
<223> Artificial sequence

<220>
<221> misc_feature
<222> (1)..(20)
<223> Forward Primer for HPV Type 56

<400> 17
gtgtataggg atgattttcc 20

<210> 18
<211> 20
<212> DNA
<213> Artificial

<220>
<223> Artificial sequence

<220>
<221> misc_feature
<222> (1)..(20)
<223> Forward Primer for HPV Type 58

<400> 18
gtgtatagag atggaaatcc 20

<210> 19
<211> 20
<212> DNA
<213> Artificial

<220>
<223> Artificial sequence

<220>
<221> misc_feature
<222> (1)..(20)
<223> Forward Primer for HPV Type 59

<400> 19
gtgtatagag actgtagacc 20

<210> 20
<211> 24
<212> DNA
<213> Artificial

<220>
<223> Artificial sequence

<220>
<221> misc_feature
<222> (1)..(24)
<223> Forward Primer for HPV Type 66

<400> 20
aactagtata tagaaacaat tggc 24

<210> 21
<211> 21
<212> DNA
<213> Artificial

<220>
<223> Artificial sequence

<220>
<221> misc_feature
<222> (1)..(21)
<223> Forward Primer for HPV Type 68

<400> 21
gtagtatata gggacggggt a                                21

<210> 22
<211> 25
<212> DNA
<213> Artificial

<220>
<223> Artificial sequence

<220>
<221> misc_feature
<222> (1)..(25)
<223> Forward Primer for HPV Type 69

<400> 22
gaatagtgta tagaaatgat agtgc                            25

<210> 23
<211> 25
<212> DNA
<213> Artificial

<220>
<223> Artificial sequence

<220>
<221> misc_feature
<222> (1)..(25)
<223> Forward Primer for HPV Type 73

<400> 23
gtattgtata tagaaaggat aaacc                            25

<210> 24
<211> 19
<212> DNA
<213> Artificial

<220>
<223> Artificial sequence

<220>
<221> misc_feature
<222> (1)..(19)
<223> Forward Primer for HPV Type 82

<400> 24
gtatatagga caatacgcc                                   19

<210> 25
<211> 23
<212> DNA
<213> Artificial

<220>
<223> Artificial sequence


<220>
<221> misc_feature
<222> (1)..(23)
<223> Backward Primer for HPV Type 6

<400> 25
agctgttgca cttctctgat gtc                    23


<210> 26
<211> 23
<212> DNA
<213> Artificial

<220>
<223> Artificial sequence


<220>
<221> misc_feature
<222> (1)..(23)
<223> Backward Primer for HPV Type 11

<400> 26
aggtcttgta gttgtctgat gtc                    23


<210> 27
<211> 24
<212> DNA
<213> Artificial

<220>
<223> Artificial sequence


<220>
<221> misc_feature
<222> (1)..(24)
<223> Backward Primer for HPV Type 16

<220>
<221> misc_feature
<222> (17)..(17)
<223> R is A or G

<220>
<221> misc_feature
<222> (21)..(21)
<223> R is A or G

<400> 27
aattgctcat aacagtrgag rtca                    24


<210> 28
<211> 24
<212> DNA
<213> Artificial

<220>
<223> Artificial sequence


<220>
<221> misc_feature
<222> (1)..(24)
<223> Backward Primer for HPV Type 18

<220>
<221> misc_feature
<222> (17)..(17)
<223> S is C or G

<400> 28
aattgctcgt gacatasaag gtca                    24


<210> 29
<211> 24
<212> DNA
<213> Artificial

<220>
<223> Artificial sequence


<220>
<221> misc_feature
<222> (1)..(24)
<223> Backward Primer for HPV Type 26

<220>
<221> misc_feature
<222> (12)..(12)
<223> R is A or G

<220>
<221> misc_feature
<222> (15)..(15)
<223> S is C or G

<220>
<221> misc_feature
<222> (16)..(16)
<223> Y is C or T

<400> 29
aattgttcgt arcasygtag gtca                    24

<210> 30
<211> 24
<212> DNA
<213> Artificial

<220>
<223> Artificial sequence


<220>
<221> misc_feature
<222> (1)..(24)
<223> Backward Primer for HPV Type 31

<220>
<221> misc_feature
<222> (17)..(17)
<223> R is A or G

<220>
<221> misc_feature
<222> (21)..(21)
<223> R is A or G

<400> 30
aattgctcat aacagtrgag rtca                                24


<210> 31
<211> 24
<212> DNA
<213> Artificial

<220>
<223> Artificial sequence


<220>
<221> misc_feature
<222> (1)..(24)
<223> Backward Primer for HPV Type 33

<220>
<221> misc_feature
<222> (12)..(12)
<223> R is A or G

<400> 31
aattgctcat arcagtatag gtca                                24


<210> 32
<211> 24
<212> DNA
<213> Artificial

<220>

<223> Artificial sequence

<220>
<221> misc_feature
<222> (1)..(24)
<223> Backward Primer for HPV Type 35

<220>
<221> misc_feature
<222> (12)..(12)
<223> R is A or G

<400> 32
aattgctcat arcagtatag gtca 24

<210> 33
<211> 24
<212> DNA
<213> Artificial

<220>
<223> Artificial sequence

<220>
<221> misc_feature
<222> (1)..(24)
<223> Backward Primer for HPV Type 39

<220>
<221> misc_feature
<222> (17)..(17)
<223> S is C or G

<400> 33
aattgctcgt gacatasaag gtca 24

<210> 34
<211> 20
<212> DNA
<213> Artificial

<220>
<223> Artificial sequence

<220>
<221> misc_feature
<222> (1)..(20)
<223> Backward Primer for HPV Type 42

<400> 34
cccaaaagca tctgttgcag 20

<210> 35

<211> 23
<212> DNA
<213> Artificial

<220>
<223> Artificial sequence


<220>
<221> misc_feature
<222> (1)..(23)
<223> Backward Primer for HPV Type 43

<400> 35
cagtcttcta gcttcttgat gtc                                    23


<210> 36
<211> 23
<212> DNA
<213> Artificial

<220>
<223> Artificial sequence


<220>
<221> misc_feature
<222> (1)..(23)
<223> Backward Primer for HPV Type 44

<400> 36
agcgtatgta ggtgatggat gtc                                    23


<210> 37
<211> 24
<212> DNA
<213> Artificial

<220>
<223> Artificial sequence


<220>
<221> misc_feature
<222> (1)..(24)
<223> Backward Primer for HPV Type 45

<220>
<221> misc_feature
<222> (12)..(12)
<223> R is A or G

<220>
<221> misc_feature
<222> (17)..(18)
<223> M is A or C

<400> 37
aattgctcgt arcacammag gtca                    24


<210> 38
<211> 24
<212> DNA
<213> Artificial

<220>
<223> Artificial sequence


<220>
<221> misc_feature
<222> (1)..(24)
<223> Backward Primer for HPV Type 51

<220>
<221> misc_feature
<222> (9)..(9)
<223> R is A or G

<220>
<221> misc_feature
<222> (11)..(11)
<223> R is A or G

<220>
<221> misc_feature
<222> (20)..(20)
<223> R is A or G

<400> 38
aattgctcrt rgcattgcar gtca                    24


<210> 39
<211> 24
<212> DNA
<213> Artificial

<220>
<223> Artificial sequence


<220>
<221> misc_feature
<222> (1)..(24)
<223> Backward Primer for HPV Type 52

<220>
<221> misc_feature
<222> (16)..(16)
<223> W is A or T

<400> 39
aattgctcat agcagwrtag gtca                    24

<210> 40
<211> 24
<212> DNA
<213> Artificial

<220>
<223> Artificial sequence


<220>
<221> misc_feature
<222> (1)..(24)
<223> Backward Primer for HPV Type 53

<220>
<221> misc_feature
<222> (9)..(9)
<223> R is A or G

<220>
<221> misc_feature
<222> (11)..(11)
<223> R is A or G

<220>
<221> misc_feature
<222> (20)..(20)
<223> R is A or G

<400> 40
aattgctcrt rgcattgcar gtca                                24


<210> 41
<211> 24
<212> DNA
<213> Artificial

<220>
<223> Artificial sequence


<220>
<221> misc_feature
<222> (1)..(24)
<223> Backward Primer for HPV Type 56

<220>
<221> misc_feature
<222> (15)..(15)
<223> Y is C or T

<400> 41
aattgctcat tgcaytgtag gtca                                24


<210> 42
<211> 24

<212> DNA
<213> Artificial

<220>
<223> Artificial sequence

<220>
<221> misc_feature
<222> (1)..(24)
<223> Backward Primer for HPV Type 58

<220>
<221> misc_feature
<222> (16)..(16)
<223> W is A or T

<220>
<221> misc_feature
<222> (17)..(17)
<223> R is A or G

<400> 42
aattgctcat agcagwrtag gtca 24

<210> 43
<211> 24
<212> DNA
<213> Artificial

<220>
<223> Artificial sequence

<220>
<221> misc_feature
<222> (1)..(24)
<223> Backward Primer for HPV Type 59

<220>
<221> misc_feature
<222> (12)..(12)
<223> R is A or G

<220>
<221> misc_feature
<222> (17)..(18)
<223> M is A or C

<400> 43
aattgctcgt arcacammag gtca 24

<210> 44
<211> 24
<212> DNA
<213> Artificial

<220>
<223> Artificial sequence

<220>
<221> misc_feature
<222> (1)..(24)
<223> Backward Primer for HPV Type 66

<220>
<221> misc_feature
<222> (15)..(15)
<223> Y is C or T

<400> 44
aattgctcat tgcaytgtag gtca 24

<210> 45
<211> 24
<212> DNA
<213> Artificial

<220>
<223> Artificial sequence

<220>
<221> misc_feature
<222> (1)..(24)
<223> Backward Primer for HPV Type 68

<400> 45
aattgctcgt gacatacaag gtcg 24

<210> 46
<211> 24
<212> DNA
<213> Artificial

<220>
<223> Artificial sequence

<220>
<221> misc_feature
<222> (1)..(24)
<223> Backward Primer for HPV Type 69

<220>
<221> misc_feature
<222> (12)..(12)
<223> R is A or G

<220>
<221> misc_feature
<222> (15)..(15)
<223> S is C or G

<220>
<221> misc_feature
<222> (16)..(16)
<223> Y is C or T

<400> 46
aattgttcgt arcasygtag gtca                                24


<210> 47
<211> 24
<212> DNA
<213> Artificial

<220>
<223> Artificial sequence


<220>
<221> misc_feature
<222> (1)..(24)
<223> Backward Primer for HPV Type 73

<400> 47
caatgactcg taacatgtaa ggtc                                24


<210> 48
<211> 24
<212> DNA
<213> Artificial

<220>
<223> Artificial sequence


<220>
<221> misc_feature
<222> (1)..(24)
<223> Backward Primer for HPV Type 82

<220>
<221> misc_feature
<222> (9)..(9)
<223> R is A or G

<220>
<221> misc_feature
<222> (11)..(11)
<223> R is A or G

<220>
<221> misc_feature
<222> (20)..(20)
<223> R is A or G

<400> 48
aattgctcrt rgcattgcar gtca                                24

<210> 49
<211> 20
<212> DNA
<213> Artificial

<220>
<223> Artificial sequence

<220>
<221> misc_feature
<222> (1)..(20)
<223> Extension Primer for HPV Type 6

<400> 49
gaagtggacg gacaagattc                                20

<210> 50
<211> 20
<212> DNA
<213> Artificial

<220>
<223> Artificial sequence

<220>
<221> misc_feature
<222> (1)..(20)
<223> Extension Primer for HPV Type 11

<400> 50
caaggtggac aacacagacg                                20

<210> 51
<211> 22
<212> DNA
<213> Artificial

<220>
<223> Artificial sequence

<220>
<221> misc_feature
<222> (1)..(22)
<223> Extension Primer for HPV Type 16

<400> 51
gcatggagat acacctacat tg                             22

<210> 52
<211> 21
<212> DNA

<213> Artificial

<220>
<223> Artificial sequence

<220>
<221> misc_feature
<222> (1)..(21)
<223> Extension Primer for HPV Type 18

<400> 52
gacaggaacg actccaacga c 21

<210> 53
<211> 20
<212> DNA
<213> Artificial

<220>
<223> Artificial sequence

<220>
<221> misc_feature
<222> (1)..(20)
<223> Extension Primer for HPV Type 26

<400> 53
gagaccaagg cgccaaacag 20

<210> 54
<211> 22
<212> DNA
<213> Artificial

<220>
<223> Artificial sequence

<220>
<221> misc_feature
<222> (1)..(22)
<223> Extension Primer for HPV Type 31

<400> 54
catgcgtgga gaaacaccta cg 22

<210> 55
<211> 21
<212> DNA
<213> Artificial

<220>
<223> Artificial sequence

<220>
<221> misc_feature
<222> (1)..(21)
<223> Extension Primer for HPV Type 33

<400> 55
cgacgtagag aaactgcact g 21

<210> 56
<211> 20
<212> DNA
<213> Artificial

<220>
<223> Artificial sequence

<220>
<221> misc_feature
<222> (1)..(20)
<223> Extension Primer for HPV Type 35

<400> 56
gacaggtcgg tgtatgtcct 20

<210> 57
<211> 21
<212> DNA
<213> Artificial

<220>
<223> Artificial sequence

<220>
<221> misc_feature
<222> (1)..(21)
<223> Extension Primer for HPV Type 39

<400> 57
gaccgcagac taacacgaag a 21

<210> 58
<211> 20
<212> DNA
<213> Artificial

<220>
<223> Artificial sequence

<220>
<221> misc_feature
<222> (1)..(20)
<223> Extension Primer for HPV Type 42

<400> 58
tgaccaagcc aaacaggaca                                20

<210> 59
<211> 20
<212> DNA
<213> Artificial

<220>
<223> Artificial sequence

<220>
<221> misc_feature
<222> (1)..(20)
<223> Extension Primer for HPV Type 43

<400> 59
ggaccagcaa gtgaatctac                                20

<210> 60
<211> 20
<212> DNA
<213> Artificial

<220>
<223> Artificial sequence

<220>
<221> misc_feature
<222> (1)..(20)
<223> Extension Primer for HPV Type 44

<400> 60
cgcaagacgt tacacagcct                                20

<210> 61
<211> 21
<212> DNA
<213> Artificial

<220>
<223> Artificial sequence

<220>
<221> misc_feature
<222> (1)..(21)
<223> Extension Primer for HPV Type 45

<400> 61
gaaagacttc gcagacgtag g                              21

<210> 62
<211> 20
<212> DNA
<213> Artificial

<220>
<223> Artificial sequence


<220>
<221> misc_feature
<222> (1)..(20)
<223> Extension Primer for HPV Type 51

<400> 62
acgtacacga caacgtaacg                                    20


<210> 63
<211> 21
<212> DNA
<213> Artificial

<220>
<223> Artificial sequence


<220>
<221> misc_feature
<222> (1)..(21)
<223> Extension Primer for HPV Type 52

<400> 63
ctgtgaccca agtgtaacgt c                                  21


<210> 64
<211> 18
<212> DNA
<213> Artificial

<220>
<223> Artificial sequence


<220>
<221> misc_feature
<222> (1)..(18)
<223> Extension Primer for HPV Type 53

<400> 64
gaccgggtcg tgcctgac                                      18


<210> 65
<211> 21
<212> DNA
<213> Artificial

<220>
<223> Artificial sequence

<220>
<221> misc_feature
<222> (1)..(21)
<223> Extension Primer for HPV Type 56

<400> 65
gagacaaaca tctagagaac c                                    21

<210> 66
<211> 19
<212> DNA
<213> Artificial

<220>
<223> Artificial sequence

<220>
<221> misc_feature
<222> (1)..(19)
<223> Extension Primer for HPV Type 58

<400> 66
gacagggcgc tgtgcagtg                                    19

<210> 67
<211> 19
<212> DNA
<213> Artificial

<220>
<223> Artificial sequence

<220>
<221> misc_feature
<222> (1)..(19)
<223> Extension Primer for HPV Type 59

<400> 67
caaagacaag cgcgtagtg                                    19

<210> 68
<211> 24
<212> DNA
<213> Artificial

<220>
<223> Artificial sequence

<220>

<221> misc_feature
<222> (1)..(24)
<223> Extension Primer for HPV Type 66

<400> 68
gacatacgag tagacaagct acag 24

<210> 69
<211> 18
<212> DNA
<213> Artificial

<220>
<223> Artificial sequence

<220>
<221> misc_feature
<222> (1)..(18)
<223> Extension Primer for HPV Type 68

<400> 69
gcagacgcac acggcagg 18

<210> 70
<211> 20
<212> DNA
<213> Artificial

<220>
<223> Artificial sequence

<220>
<221> misc_feature
<222> (1)..(20)
<223> Extension Primer for HPV Type 69

<400> 70
ggatgaaaag cgacggttcc 20

<210> 71
<211> 23
<212> DNA
<213> Artificial

<220>
<223> Artificial sequence

<220>
<221> misc_feature
<222> (1)..(23)
<223> Extension Primer for HPV Type 73

<400> 71

cggtttcatc aaatagcaga aca 23

<210> 72
<211> 22
<212> DNA
<213> Artificial

<220>
<223> Artificial sequence

<220>
<221> misc_feature
<222> (1)..(22)
<223> Extension Primer for HPV Type 82

<400> 72
gtgaaaccca ggtgtaataa cg 22

<210> 73
<211> 20
<212> DNA
<213> Artificial

<220>
<223> Artificial Sequence

<220>
<221> misc_feature
<222> (1)..(20)
<223> Forward primer for HPV Type 16

<400> 73
gtatatagag atgggaatcc 20

<210> 74
<211> 20
<212> DNA
<213> Artificial

<220>
<223> Artificial sequence

<220>
<221> misc_feature
<222> (1)..(20)
<223> Forward Primer for HPV Type 33

<400> 74
gtatatagag agggaaatcc 20

<210> 75
<211> 24

<212> DNA
<213> Artificial

<220>
<223> Artificial sequence


<220>
<221> misc_feature
<222> (1)..(24)
<223> Backward primer for HPV Type 16

<400> 75
aattgctcat aacagtagag atca                                24


<210> 76
<211> 24
<212> DNA
<213> Artificial

<220>
<223> Artificial Sequence


<220>
<221> misc_feature
<222> (1)..(24)
<223> Backward primer for HPV Type 18

<400> 76
aattgctcgt gacatagaag gtca                                24


<210> 77
<211> 24
<212> DNA
<213> Artificial

<220>
<223> Artificial sequence


<220>
<221> misc_feature
<222> (1)..(24)
<223> Backward Primer for HPV Type 26

<400> 77
aattgttcgt agcagcgtag gtca                                24


<210> 78
<211> 24
<212> DNA
<213> Artificial

<220>
<223> Artificial sequence

<220>
<221> misc_feature
<222> (1)..(24)
<223> backward primer for HPV type 31

<400> 78
aattgctcat aacagtggag gtca                                24

<210> 79
<211> 24
<212> DNA
<213> Artificial

<220>
<223> Artificial sequence

<220>
<221> misc_feature
<222> (1)..(24)
<223> Backward primer for HPV type 33

<400> 79
aattgctcat agcagtatag gtca                                24

<210> 80
<211> 24
<212> DNA
<213> Artificial

<220>
<223> Artificial sequence

<220>
<221> misc_feature
<222> (1)..(24)
<223> Backward Primer for HPV Type 35

<400> 80
aattgctcat aacagtatag gtca                                24

<210> 81
<211> 24
<212> DNA
<213> Artificial

<220>
<223> Artificial sequence

<220>
<221> misc_feature
<222> (1)..(24)

<223> Backward primer for HPV Type 39

<400> 81
aattgctcgt gacatacaag gtca                                    24


<210> 82
<211> 24
<212> DNA
<213> Artificial

<220>
<223> Artificial sequence


<220>
<221> misc_feature
<222> (1)..(24)
<223> Backward Primer for HPV Type 45

<400> 82
aattgctcgt aacacaacag gtca                                    24


<210> 83
<211> 24
<212> DNA
<213> Artificial

<220>
<223> Artificial sequence


<220>
<221> misc_feature
<222> (1)..(24)
<223> Backward primer for HPV type 51

<400> 83
aattgctcgt agcattgcaa gtca                                    24


<210> 84
<211> 24
<212> DNA
<213> Artificial

<220>
<223> Artificial sequence


<220>
<221> misc_feature
<222> (1)..(24)
<223> Backward primer for HPV type 52

<400> 84
aattgctcat agcagtgtag gtca                                    24

<210> 85
<211> 24
<212> DNA
<213> Artificial

<220>
<223> Artificial sequence

<220>
<221> misc_feature
<222> (1)..(24)
<223> Backward primer for HPV type 53

<400> 85
aattgctcat ggcattgcag gtca 24

<210> 86
<211> 24
<212> DNA
<213> Artificial

<220>
<223> Artificial sequence

<220>
<221> misc_feature
<222> (1)..(24)
<223> Backward primer for HPV type 56

<400> 86
aattgctcat tgcactgtag gtca 24

<210> 87
<211> 24
<212> DNA
<213> Artificial

<220>
<223> Artificial sequence

<220>
<221> misc_feature
<222> (1)..(24)
<223> Backward primer for HPV type 58

<400> 87
aattgctcat agcagaatag gtca 24

<210> 88
<211> 24
<212> DNA
<213> Artificial

<220>
<223> Artificial sequence

<220>
<221> misc_feature
<222> (1)..(24)
<223> Backward primer for HPV type 59

<400> 88
aattgctcgt agcacacaag gtca 24

<210> 89
<211> 24
<212> DNA
<213> Artificial

<220>
<223> Artificial sequence

<220>
<221> misc_feature
<222> (1)..(24)
<223> Backward primer for HPV type 66

<400> 89
aattgctcat tgcattgtag gtca 24

<210> 90
<211> 24
<212> DNA
<213> Artificial

<220>
<223> Artificial sequence

<220>
<221> misc_feature
<222> (1)..(24)
<223> Backward primer for HPV type 69

<400> 90
aattgttcgt aacactgtag gtca 24

<210> 91
<211> 24
<212> DNA
<213> Artificial

<220>
<223> Artificial sequence

<220>
<221> misc_feature
<222> (1)..(24)
<223> Backward primer for HPV type 82

<400> 91

aattgctcgt agcattgcaa gtca 24

**TABLE 1A**

| **HPV** | **SEQ ID.** | **Upstream Primer** | **SEQ ID.** | **Downstream Primer ID.** | **SEQ** | **Extension Primer** |
|---|---|---|---|---|---|---|
| 6 | A1 | GCAACAACAGTTGAAGAAGAAAC | B1 | AGCTGTTGCACTTCTCTGATGTC | C1 | GAAGTGGACGGACAAGATTC |
| 11 | A2 | GCACCTACAGTAGAAGAAGAAAC | B2 | AGGTCTTGTAGTTGTCTGATGTC | C2 | CAAGGTGGACAACACAGACG |
| 16 | A3 | GTATATAGAGAKGGRAATCC | B3 | AATTGCTCATAACAGTRGAGRTCA | C3 | GCATGGAGATACACCTACATTG |
| 18 | A4 | GTGTATAGAGACAGTATACCG | B4 | AATTGCTCGTGACATASAAGGTCA | C4 | GACAGGAACGACTCCAACGAC |
| 26 | A5 | GTATATAGAGATAGGAGTCC | B5 | AATTGTTCGTARCASYGTAGGTCA | C5 | GAGACCAAGGCGCCAAACAG |
| 31 | A6 | GTATATAGGGACGACACACC | B6 | AATTGCTCATAACAGTRGAGRTCA | C6 | CATGCGTGGAGAAACACCTACG |
| 33 | A7 | GTATATAGAGAKGGRAATCC | B7 | AATTGCTCATARCAGTATAGGTCA | C7 | CGACGTAGAGAAACTGCACTG |
| 35 | A8 | GTATATAGAGAAGGCCAGCC | B8 | AATTGCTCATARCAGTATAGGTCA | C8 | GACAGGTCGGTGTATGTCCT |
| 39 | A9 | GTATATAGGGACGGGGAACC | B9 | AATTGCTCGTGACATASAAGGTCA | C9 | GACCGCAGACTAACACGAAGA |
| 42 | A10 | TGGTATACAGTGGAGAAAGAAAC | B10 | CCCAAAAGCATCTGTTGCAG | C10 | TGACCAAGCCAAACAGGACA |
| 43 | A11 | GCAGATACTGTAGAAGAAGAAAC | B11 | CAGTCTTCTAGCTTCTTGATGTC | C11 | GGACCAGCAAGTGAATCTAC |
| 44 | A12 | GCAGTAACAGTGGAAGAAGAAAC | B12 | AGCGTATGTAGGTGATGGATGTC | C12 | CGCAAGACGTTACACAGCCT |
| 45 | A13 | GTGTATAGAGACTGTATAGC | B13 | AATTGCTCGTARCACAMMAGGTCA | C13 | GAAAGACTTCGCAGACGTAGG |
| 51 | A14 | GATTGTATATAGGGATAATAATCCAT | B14 | AATTGCTCRTRGCATTGCARGTCA | C14 | ACGTACACGACAACGTAACG |
| 52 | A15 | GAATAGTATATAGAGACAATAATCC | B15 | AATTGCTCATAGCAGWRTAGGTCA | C15 | CTGTGACCCAAGTGTAACGTC |
| 53 | A16 | GTGTATAGAGACGGGTATCC | B16 | AATTGCTCRTRGCATTGCARGTCA | C16 | GACCGGGTCGTGCCTGAC |
| 56 | A17 | GTGTATAGGGATGATTTTCC | B17 | AATTGCTCATTGCAYTGTAGGTCA | C17 | GAGACAAACATCTAGAGAACC |
| 58 | A18 | GTGTATAGAGATGGAAATCC | B18 | AATTGCTCATAGCAGWRTAGGTCA | C18 | GACAGGGCGCTGTGCAGTG |
| 59 | A19 | GTGTATAGAGACTGTAGACC | B19 | AATTGCTCGTARCACAMMAGGTCA | C19 | CAAAGACAAGCGCGTAGTG |
| 66 | A20 | AACTAGTATATAGAAACAATTGGC | B20 | AATTGCTCATTGCAYTGTAGGTCA | C20 | GACATACGAGTAGACAAGCTACAG |
| 68 | A21 | GTAGTATATAGGGACGGGGTA | B21 | AATTGCTCGTGACATACAAGGTCG | C21 | GCAGACGCACACGGCAGG |
| 69 | A22 | GAATAGTGTATAGAAATGATAGTGC | B22 | AATTGTTCGTARCASYGTAGGTCA | C22 | GGATGAAAAGCGACGGTTCC |
| 73 | A23 | GTATTGTATATAGAAAGGATAAACC | B23 | CAATGACTCGTAACATGTAAGGTC | C23 | CGGTTTCATCAAATAGCAGAACA |
| 82 | A24 | GTATATAGGACAATACGCC | B24 | AATTGCTCRTRGCATTGCARGTCA | C24 | GTGAAACCCAGGTGTAATAACG |

**TABLE 1B**

| **HPV** | **SEQ** | **Upstream Primer ID.** | **SEQ ID.** | **Downstream Primer ID.** | **SEQ** | **Extension Primer** |
|---|---|---|---|---|---|---|
| 16 | A25 | GTATATAGAGATGGGAATCC | B25 | AATTGCTCATAACAGTAGAGATCA | C3 | GCATGGAGATACACCTACATTG |
| 18 | A4 | GTGTATAGAGACAGTATACCG | B26 | AATTGCTCGTGACATAGAAGGTCA | C4 | GACAGGAACGACTCCAACGAC |
| 26 | A5 | GTATATAGAGATAGGAGTCC | B27 | AATTGTTCGTAGCAGCGTAGGTCA | C5 | GAGACCAAGGCGCCAAACAG |
| 31 | A6 | GTATATAGGGACGACACACC | B28 | AATTGCTCATAACAGTGGAGGTCA | C6 | CATGCGTGGAGAAACACCTACG |
| 33 | A26 | GTATATAGAGAGGGAAATCC | B29 | AATTGCTCATAGCAGTATAGGTCA | C7 | CGACGTAGAGAAACTGCACTG |
| 35 | A8 | GTATATAGAGAAGGCCAGCC | B30 | AATTGCTCATAACAGTATAGGTCA | C8 | GACAGGTCGGTGTATGTCCT |
| 39 | A9 | GTATATAGGGACGGGGAACC | B31 | AATTGCTCGTGACATACAAGGTCA | C9 | GACCGCAGACTAACACGAAGA |
| 45 | A13 | GTGTATAGAGACTGTATAGC | B32 | AATTGCTCGTAACACAACAGGTCA | C13 | GAAAGACTTCGCAGACGTAGG |
| 51 | A14 | GATTGTATATAGGGATAATAATCCAT | B33 | AATTGCTCGTAGCATTGCAAGTCA | C14 | ACGTACACGACAACGTAACG |
| 52 | A15 | GAATAGTATATAGAGACAATAATCC | B34 | AATTGCTCATAGCAGTGTAGGTCA | C15 | CTGTGACCCAAGTGTAACGTC |
| 53 | A16 | GTGTATAGAGACGGGTATCC | B35 | AATTGCTCATGGCATTGCAGGTCA | C16 | GACCGGGTCGTGCCTGAC |
| 56 | A17 | GTGTATAGGGATGATTTTCC | B36 | AATTGCTCATTGCACTGTAGGTCA | C17 | GAGACAAACATCTAGAGAACC |
| 58 | A18 | GTGTATAGAGATGGAAATCC | B37 | AATTGCTCATAGCAGAATAGGTCA | C18 | GACAGGGCGCTGTGCAGTG |
| 59 | A19 | GTGTATAGAGACTGTAGACC | B38 | AATTGCTCGTAGCACACAAGGTCA | C19 | CAAAGACAAGCGCGTAGTG |
| 66 | A20 | AACTAGTATATAGAAACAATTGGC | B39 | AATTGCTCATTGCATTGTAGGTCA | C20 | GACATACGAGTAGACAAGCTACAG |
| 69 | A22 | GAATAGTGTATAGAAATGATAGTGC | B40 | AATTGTTCGTAACACTGTAGGTCA | C22 | GGATGAAAAGCGACGGTTCC |
| 82 | A24 | GTATATAGGACAATACGCC | B41 | AATTGCTCGTAGCATTGCAAGTCA | C24 | GTGAAACCCAGGTGTAATAACG |

SEQUENCE LISTING

<110>  Autogenomics

<120>  MULTIPLEXED HPV NUCLEIC ACID ANALYSIS WITH IMPROVED SPECIFICITY

<130>  100788.0023PCT

<150>  US 60/532681
<151>  2003-12-23

<150>  US 60/556737
<151>  2004-03-26

<160>  91

<170>  PatentIn version 3.2

<210>  1
<211>  23
<212>  DNA
<213>  Artificial

<220>
<223>  Artificial sequence;  IUPAC ambiguity code applies for all
       nucleotides other than A, C, G, and T.


<220>
<221>  misc_feature
<222>  (1)..(23)
<223>  Forward Primer for HPV Type 6

<400>  1
gcaacaacag ttgaagaaga aac                                              23


<210>  2
<211>  23
<212>  DNA
<213>  Artificial

<220>
<223>  Artificial sequence;  IUPAC ambiguity code applies for all
       nucleotides other than A, C, G, and T.


<220>
<221>  misc_feature
<222>  (1)..(23)
<223>  Forward Primer for HPV Type 11

<400>  2
gcacctacag tagaagaaga aac                                              23


<210>  3
<211>  20
<212>  DNA
<213>  Artificial

<220>
<223>  Artificial sequence

<220>
<221>  misc_feature
<222>  (1)..(20)
<223>  Forward Primer for HPV Type 16

<220>
<221>  misc_feature
<222>  (12)..(12)
<223>  K is G or T

<220>
<221>  misc_feature
<222>  (15)..(15)
<223>  R is A or G

<400>  3
gtatatagag akggraatcc                                                    20


<210>  4
<211>  21
<212>  DNA
<213>  Artificial

<220>
<223>  Artificial sequence


<220>
<221>  misc_feature
<222>  (1)..(21)
<223>  Forward Primer for HPV Type 18

<400>  4
gtgtatagag acagtatacc g                                                  21


<210>  5
<211>  20
<212>  DNA
<213>  Artificial

<220>
<223>  Artificial sequence


<220>
<221>  misc_feature
<222>  (1)..(20)
<223>  Forward Primer for HPV Type 26

<400>  5
gtatatagag ataggagtcc                                                    20


<210>  6
<211>  20
<212>  DNA
<213>  Artificial

<220>

<223>  Artificial sequence

<220>
<221>  misc_feature
<222>  (1)..(20)
<223>  Forward Primer for HPV Type 31

<400>  6
gtatataggg acgacacacc                                                    20

<210>  7
<211>  20
<212>  DNA
<213>  Artificial

<220>
<223>  Artificial sequence

<220>
<221>  misc_feature
<222>  (1)..(20)
<223>  Forward Primer for HPV Type 33

<220>
<221>  misc_feature
<222>  (12)..(12)
<223>  K is G or T

<220>
<221>  misc_feature
<222>  (15)..(15)
<223>  R is A or G

<400>  7
gtatatagag akggraatcc                                                    20

<210>  8
<211>  20
<212>  DNA
<213>  Artificial

<220>
<223>  Artificial sequence

<220>
<221>  misc_feature
<222>  (1)..(20)
<223>  Forward Primer for HPV Type 35

<400>  8
gtatatagag aaggccagcc                                                    20

<210>  9
<211>  20
<212>  DNA
<213>  Artificial

<220>
<223>  Artificial sequence


<220>
<221>  misc_feature
<222>  (1)..(20)
<223>  Forward Primer for HPV Type 39

<400>  9
gtatataggg acggggaacc                                                    20


<210>  10
<211>  23
<212>  DNA
<213>  Artificial

<220>
<223>  Artificial sequence


<220>
<221>  misc_feature
<222>  (1)..(23)
<223>  Forward Primer for HPV Type 42

<400>  10
tggtatacag tggagaaaga aac                                                23


<210>  11
<211>  23
<212>  DNA
<213>  Artificial

<220>
<223>  Artificial sequence


<220>
<221>  misc_feature
<222>  (1)..(23)
<223>  Forward Primer for HPV Type 43

<400>  11
gcagatactg tagaagaaga aac                                                23


<210>  12
<211>  23
<212>  DNA
<213>  Artificial

<220>
<223>  Artificial sequence


<220>
<221>  misc_feature
<222>  (1)..(23)
<223>  Forward Primer for HPV Type 44

<400>  12
gcagtaacag tggaagaaga aac                                              23


<210>  13
<211>  20
<212>  DNA
<213>  Artificial

<220>
<223>  Artificial sequence


<220>
<221>  misc_feature
<222>  (1)..(20)
<223>  Forward Primer for HPV Type 45

<400>  13
gtgtatagag actgtatagc                                                  20


<210>  14
<211>  26
<212>  DNA
<213>  Artificial

<220>
<223>  Artificial sequence


<220>
<221>  misc_feature
<222>  (1)..(26)
<223>  Forward Primer for HPV Type 51

<400>  14
gattgtatat agggataata atccat                                           26


<210>  15
<211>  25
<212>  DNA
<213>  Artificial

<220>
<223>  Artificial sequence


<220>
<221>  misc_feature
<222>  (1)..(25)
<223>  Forward Primer for HPV Type 52

<400>  15
gaatagtata tagagacaat aatcc                                            25


<210>  16
<211>  20
<212>  DNA
<213>  Artificial

<220>
<223>  Artificial sequence


<220>
<221>  misc_feature
<222>  (1)..(20)
<223>  Forward Primer for HPV Type 53

<400>  16
gtgtatagag acgggtatcc                                                    20


<210>  17
<211>  20
<212>  DNA
<213>  Artificial

<220>
<223>  Artificial sequence


<220>
<221>  misc_feature
<222>  (1)..(20)
<223>  Forward Primer for HPV Type 56

<400>  17
gtgtataggg atgattttcc                                                    20


<210>  18
<211>  20
<212>  DNA
<213>  Artificial

<220>
<223>  Artificial sequence


<220>
<221>  misc_feature
<222>  (1)..(20)
<223>  Forward Primer for HPV Type 58

<400>  18
gtgtatagag atggaaatcc                                                    20


<210>  19
<211>  20
<212>  DNA
<213>  Artificial

<220>
<223>  Artificial sequence


<220>
<221>  misc_feature
<222>  (1)..(20)
<223>  Forward Primer for HPV Type 59

<400>  19
gtgtatagag actgtagacc                                                    20


<210>  20
<211>  24
<212>  DNA
<213>  Artificial

<220>
<223>  Artificial sequence


<220>
<221>  misc_feature
<222>  (1)..(24)
<223>  Forward Primer for HPV Type 66

<400>  20
aactagtata tagaaacaat tggc                                               24


<210>  21
<211>  21
<212>  DNA
<213>  Artificial

<220>
<223>  Artificial sequence


<220>
<221>  misc_feature
<222>  (1)..(21)
<223>  Forward Primer for HPV Type 68

<400>  21
gtagtatata gggacggggt a                                                  21


<210>  22
<211>  25
<212>  DNA
<213>  Artificial

<220>
<223>  Artificial sequence


<220>
<221>  misc_feature
<222>  (1)..(25)
<223>  Forward Primer for HPV Type 69

<400>  22
gaatagtgta tagaaatgat agtgc                                              25


<210>  23
<211>  25
<212>  DNA
<213>  Artificial

<220>
<223>  Artificial sequence


<220>
<221>  misc_feature
<222>  (1)..(25)
<223>  Forward Primer for HPV Type 73

<400>  23
gtattgtata tagaaaggat aaacc                                          25


<210>  24
<211>  19
<212>  DNA
<213>  Artificial

<220>
<223>  Artificial sequence


<220>
<221>  misc_feature
<222>  (1)..(19)
<223>  Forward Primer for HPV Type 82

<400>  24
gtatatagga caatacgcc                                                 19


<210>  25
<211>  23
<212>  DNA
<213>  Artificial

<220>
<223>  Artificial sequence


<220>
<221>  misc_feature
<222>  (1)..(23)
<223>  Backward Primer for HPV Type 6

<400>  25
agctgttgca cttctctgat gtc                                            23


<210>  26
<211>  23
<212>  DNA
<213>  Artificial

<220>
<223>  Artificial sequence


<220>
<221>  misc_feature
<222>  (1)..(23)
<223>  Backward Primer for HPV Type 11

<400>  26
aggtcttgta gttgtctgat gtc                                              23


<210>  27
<211>  24
<212>  DNA
<213>  Artificial

<220>
<223>  Artificial sequence


<220>
<221>  misc_feature
<222>  (1)..(24)
<223>  Backward Primer for HPV Type 16

<220>
<221>  misc_feature
<222>  (17)..(17)
<223>  R is A or G

<220>
<221>  misc_feature
<222>  (21)..(21)
<223>  R is A or G

<400>  27
aattgctcat aacagtrgag rtca                                             24


<210>  28
<211>  24
<212>  DNA
<213>  Artificial

<220>
<223>  Artificial sequence


<220>
<221>  misc_feature
<222>  (1)..(24)
<223>  Backward Primer for HPV Type 18

<220>
<221>  misc_feature
<222>  (17)..(17)
<223>  S is C or G

<400>  28
aattgctcgt gacatasaag gtca                                             24


<210>  29
<211>  24
<212>  DNA
<213>  Artificial

<220>
<223>  Artificial sequence

<220>
<221>  misc_feature
<222>  (1)..(24)
<223>  Backward Primer for HPV Type 26

<220>
<221>  misc_feature
<222>  (12)..(12)
<223>  R is A or G

<220>
<221>  misc_feature
<222>  (15)..(15)
<223>  S is C or G

<220>
<221>  misc_feature
<222>  (16)..(16)
<223>  Y is C or T

<400>  29
aattgttcgt arcasygtag gtca                                              24


<210>  30
<211>  24
<212>  DNA
<213>  Artificial

<220>
<223>  Artificial sequence


<220>
<221>  misc_feature
<222>  (1)..(24)
<223>  Backward Primer for HPV Type 31

<220>
<221>  misc_feature
<222>  (17)..(17)
<223>  R is A or G

<220>
<221>  misc_feature
<222>  (21)..(21)
<223>  R is A or G

<400>  30
aattgctcat aacagtrgag rtca                                              24


<210>  31
<211>  24
<212>  DNA
<213>  Artificial

<220>
<223>  Artificial sequence


<220>

<221>  misc_feature
<222>  (1)..(24)
<223>  Backward Primer for HPV Type 33

<220>
<221>  misc_feature
<222>  (12)..(12)
<223>  R is A or G

<400>  31
aattgctcat arcagtatag gtca                                              24


<210>  32
<211>  24
<212>  DNA
<213>  Artificial

<220>
<223>  Artificial sequence


<220>
<221>  misc_feature
<222>  (1)..(24)
<223>  Backward Primer for HPV Type 35

<220>
<221>  misc_feature
<222>  (12)..(12)
<223>  R is A or G

<400>  32
aattgctcat arcagtatag gtca                                              24


<210>  33
<211>  24
<212>  DNA
<213>  Artificial

<220>
<223>  Artificial sequence


<220>
<221>  misc_feature
<222>  (1)..(24)
<223>  Backward Primer for HPV Type 39

<220>
<221>  misc_feature
<222>  (17)..(17)
<223>  S is C or G

<400>  33
aattgctcgt gacatasaag gtca                                              24


<210>  34
<211>  20
<212>  DNA
<213>  Artificial

<220>
<223>  Artificial sequence


<220>
<221>  misc_feature
<222>  (1)..(20)
<223>  Backward Primer for HPV Type 42

<400>  34
cccaaaagca tctgttgcag                                                    20


<210>  35
<211>  23
<212>  DNA
<213>  Artificial

<220>
<223>  Artificial sequence


<220>
<221>  misc_feature
<222>  (1)..(23)
<223>  Backward Primer for HPV Type 43

<400>  35
cagtcttcta gcttcttgat gtc                                                23


<210>  36
<211>  23
<212>  DNA
<213>  Artificial

<220>
<223>  Artificial sequence


<220>
<221>  misc_feature
<222>  (1)..(23)
<223>  Backward Primer for HPV Type 44

<400>  36
agcgtatgta ggtgatggat gtc                                                23


<210>  37
<211>  24
<212>  DNA
<213>  Artificial

<220>
<223>  Artificial sequence


<220>
<221>  misc_feature
<222>  (1)..(24)
<223>  Backward Primer for HPV Type 45

<220>
<221>  misc_feature
<222>  (12)..(12)
<223>  R is A or G

<220>
<221>  misc_feature
<222>  (17)..(18)
<223>  M is A or C

<400>  37
aattgctcgt arcacammag gtca                                              24


<210>  38
<211>  24
<212>  DNA
<213>  Artificial

<220>
<223>  Artificial sequence


<220>
<221>  misc_feature
<222>  (1)..(24)
<223>  Backward Primer for HPV Type 51

<220>
<221>  misc_feature
<222>  (9)..(9)
<223>  R is A or G

<220>
<221>  misc_feature
<222>  (11)..(11)
<223>  R is A or G

<220>
<221>  misc_feature
<222>  (20)..(20)
<223>  R is A or G

<400>  38
aattgctcrt rgcattgcar gtca                                              24


<210>  39
<211>  24
<212>  DNA
<213>  Artificial

<220>
<223>  Artificial sequence


<220>
<221>  misc_feature
<222>  (1)..(24)
<223>  Backward Primer for HPV Type 52

<220>

<221>  misc_feature
<222>  (16)..(16)
<223>  W is A or T

<400>  39
aattgctcat agcagwrtag gtca                                              24


<210>  40
<211>  24
<212>  DNA
<213>  Artificial

<220>
<223>  Artificial sequence


<220>
<221>  misc_feature
<222>  (1)..(24)
<223>  Backward Primer for HPV Type 53

<220>
<221>  misc_feature
<222>  (9)..(9)
<223>  R is A or G

<220>
<221>  misc_feature
<222>  (11)..(11)
<223>  R is A or G

<220>
<221>  misc_feature
<222>  (20)..(20)
<223>  R is A or G

<400>  40
aattgctcrt rgcattgcar gtca                                              24


<210>  41
<211>  24
<212>  DNA
<213>  Artificial

<220>
<223>  Artificial sequence


<220>
<221>  misc_feature
<222>  (1)..(24)
<223>  Backward Primer for HPV Type 56

<220>
<221>  misc_feature
<222>  (15)..(15)
<223>  Y is C or T

<400>  41
aattgctcat tgcaytgtag gtca                                              24

<210>  42
<211>  24
<212>  DNA
<213>  Artificial

<220>
<223>  Artificial sequence


<220>
<221>  misc_feature
<222>  (1)..(24)
<223>  Backward Primer for HPV Type 58

<220>
<221>  misc_feature
<222>  (16)..(16)
<223>  W is A or T

<220>
<221>  misc_feature
<222>  (17)..(17)
<223>  R is A or G

<400>  42
aattgctcat agcagwrtag gtca                                              24


<210>  43
<211>  24
<212>  DNA
<213>  Artificial

<220>
<223>  Artificial sequence


<220>
<221>  misc_feature
<222>  (1)..(24)
<223>  Backward Primer for HPV Type 59

<220>
<221>  misc_feature
<222>  (12)..(12)
<223>  R is A or G

<220>
<221>  misc_feature
<222>  (17)..(18)
<223>  M is A or C

<400>  43
aattgctcgt arcacammag gtca                                              24


<210>  44
<211>  24
<212>  DNA
<213>  Artificial

<220>

<223>  Artificial sequence

<220>
<221>  misc_feature
<222>  (1)..(24)
<223>  Backward Primer for HPV Type 66

<220>
<221>  misc_feature
<222>  (15)..(15)
<223>  Y is C or T

<400>  44
aattgctcat tgcaytgtag gtca                                              24

<210>  45
<211>  24
<212>  DNA
<213>  Artificial

<220>
<223>  Artificial sequence

<220>
<221>  misc_feature
<222>  (1)..(24)
<223>  Backward Primer for HPV Type 68

<400>  45
aattgctcgt gacatacaag gtcg                                              24

<210>  46
<211>  24
<212>  DNA
<213>  Artificial

<220>
<223>  Artificial sequence

<220>
<221>  misc_feature
<222>  (1)..(24)
<223>  Backward Primer for HPV Type 69

<220>
<221>  misc_feature
<222>  (12)..(12)
<223>  R is A or G

<220>
<221>  misc_feature
<222>  (15)..(15)
<223>  S is C or G

<220>
<221>  misc_feature
<222>  (16)..(16)
<223>  Y is C or T

<400>  46
aattgttcgt arcasygtag gtca                                              24


<210>  47
<211>  24
<212>  DNA
<213>  Artificial

<220>
<223>  Artificial sequence


<220>
<221>  misc_feature
<222>  (1)..(24)
<223>  Backward Primer for HPV Type 73

<400>  47
caatgactcg taacatgtaa ggtc                                              24


<210>  48
<211>  24
<212>  DNA
<213>  Artificial

<220>
<223>  Artificial sequence


<220>
<221>  misc_feature
<222>  (1)..(24)
<223>  Backward Primer for HPV Type 82

<220>
<221>  misc_feature
<222>  (9)..(9)
<223>  R is A or G

<220>
<221>  misc_feature
<222>  (11)..(11)
<223>  R is A or G

<220>
<221>  misc_feature
<222>  (20)..(20)
<223>  R is A or G

<400>  48
aattgctcrt rgcattgcar gtca                                              24


<210>  49
<211>  20
<212>  DNA
<213>  Artificial

<220>
<223>  Artificial sequence

<220>
<221>  misc_feature
<222>  (1)..(20)
<223>  Extension Primer for HPV Type 6

<400>  49
gaagtggacg gacaagattc                                                    20


<210>  50
<211>  20
<212>  DNA
<213>  Artificial

<220>
<223>  Artificial sequence


<220>
<221>  misc_feature
<222>  (1)..(20)
<223>  Extension Primer for HPV Type 11

<400>  50
caaggtggac aacacagacg                                                    20


<210>  51
<211>  22
<212>  DNA
<213>  Artificial

<220>
<223>  Artificial sequence


<220>
<221>  misc_feature
<222>  (1)..(22)
<223>  Extension Primer for HPV Type 16

<400>  51
gcatggagat acacctacat tg                                                 22


<210>  52
<211>  21
<212>  DNA
<213>  Artificial

<220>
<223>  Artificial sequence


<220>
<221>  misc_feature
<222>  (1)..(21)
<223>  Extension Primer for HPV Type 18

<400>  52
gacaggaacg actccaacga c                                                  21

<210>  53
<211>  20
<212>  DNA
<213>  Artificial

<220>
<223>  Artificial sequence


<220>
<221>  misc_feature
<222>  (1)..(20)
<223>  Extension Primer for HPV Type 26

<400>  53
gagaccaagg cgccaaacag                                                    20


<210>  54
<211>  22
<212>  DNA
<213>  Artificial

<220>
<223>  Artificial sequence


<220>
<221>  misc_feature
<222>  (1)..(22)
<223>  Extension Primer for HPV Type 31

<400>  54
catgcgtgga gaaacaccta cg                                                 22


<210>  55
<211>  21
<212>  DNA
<213>  Artificial

<220>
<223>  Artificial sequence


<220>
<221>  misc_feature
<222>  (1)..(21)
<223>  Extension Primer for HPV Type 33

<400>  55
cgacgtagag aaactgcact g                                                  21


<210>  56
<211>  20
<212>  DNA
<213>  Artificial

<220>
<223>  Artificial sequence

<220>
<221>  misc_feature
<222>  (1)..(20)
<223>  Extension Primer for HPV Type 35

<400>  56
gacaggtcgg tgtatgtcct                                                    20


<210>  57
<211>  21
<212>  DNA
<213>  Artificial

<220>
<223>  Artificial sequence


<220>
<221>  misc_feature
<222>  (1)..(21)
<223>  Extension Primer for HPV Type 39

<400>  57
gaccgcagac taacacgaag a                                                  21


<210>  58
<211>  20
<212>  DNA
<213>  Artificial

<220>
<223>  Artificial sequence


<220>
<221>  misc_feature
<222>  (1)..(20)
<223>  Extension Primer for HPV Type 42

<400>  58
tgaccaagcc aaacaggaca                                                    20


<210>  59
<211>  20
<212>  DNA
<213>  Artificial

<220>
<223>  Artificial sequence


<220>
<221>  misc_feature
<222>  (1)..(20)
<223>  Extension Primer for HPV Type 43

<400>  59
ggaccagcaa gtgaatctac                                                    20

<210>  60
<211>  20
<212>  DNA
<213>  Artificial

<220>
<223>  Artificial sequence


<220>
<221>  misc_feature
<222>  (1)..(20)
<223>  Extension Primer for HPV Type 44

<400>  60
cgcaagacgt tacacagcct                                                    20


<210>  61
<211>  21
<212>  DNA
<213>  Artificial

<220>
<223>  Artificial sequence


<220>
<221>  misc_feature
<222>  (1)..(21)
<223>  Extension Primer for HPV Type 45

<400>  61
gaaagacttc gcagacgtag g                                                  21


<210>  62
<211>  20
<212>  DNA
<213>  Artificial

<220>
<223>  Artificial sequence


<220>
<221>  misc_feature
<222>  (1)..(20)
<223>  Extension Primer for HPV Type 51

<400>  62
acgtacacga caacgtaacg                                                    20


<210>  63
<211>  21
<212>  DNA
<213>  Artificial

<220>
<223>  Artificial sequence

<220>
<221>  misc_feature
<222>  (1)..(21)
<223>  Extension Primer for HPV Type 52

<400>  63
ctgtgaccca agtgtaacgt c                                                21


<210>  64
<211>  18
<212>  DNA
<213>  Artificial

<220>
<223>  Artificial sequence


<220>
<221>  misc_feature
<222>  (1)..(18)
<223>  Extension Primer for HPV Type 53

<400>  64
gaccgggtcg tgcctgac                                                    18


<210>  65
<211>  21
<212>  DNA
<213>  Artificial

<220>
<223>  Artificial sequence


<220>
<221>  misc_feature
<222>  (1)..(21)
<223>  Extension Primer for HPV Type 56

<400>  65
gagacaaaca tctagagaac c                                                21


<210>  66
<211>  19
<212>  DNA
<213>  Artificial

<220>
<223>  Artificial sequence


<220>
<221>  misc_feature
<222>  (1)..(19)
<223>  Extension Primer for HPV Type 58

<400>  66
gacagggcgc tgtgcagtg                                                   19

<210>  67
<211>  19
<212>  DNA
<213>  Artificial

<220>
<223>  Artificial sequence


<220>
<221>  misc_feature
<222>  (1)..(19)
<223>  Extension Primer for HPV Type 59

<400>  67
caaagacaag cgcgtagtg                                                    19


<210>  68
<211>  24
<212>  DNA
<213>  Artificial

<220>
<223>  Artificial sequence


<220>
<221>  misc_feature
<222>  (1)..(24)
<223>  Extension Primer for HPV Type 66

<400>  68
gacatacgag tagacaagct acag                                              24


<210>  69
<211>  18
<212>  DNA
<213>  Artificial

<220>
<223>  Artificial sequence


<220>
<221>  misc_feature
<222>  (1)..(18)
<223>  Extension Primer for HPV Type 68

<400>  69
gcagacgcac acggcagg                                                     18


<210>  70
<211>  20
<212>  DNA
<213>  Artificial

<220>
<223>  Artificial sequence

<220>
<221>  misc_feature
<222>  (1)..(20)
<223>  Extension Primer for HPV Type 69

<400>  70
ggatgaaaag cgacggttcc                                                    20


<210>  71
<211>  23
<212>  DNA
<213>  Artificial

<220>
<223>  Artificial sequence


<220>
<221>  misc_feature
<222>  (1)..(23)
<223>  Extension Primer for HPV Type 73

<400>  71
cggtttcatc aaatagcaga aca                                                23


<210>  72
<211>  22
<212>  DNA
<213>  Artificial

<220>
<223>  Artificial sequence


<220>
<221>  misc_feature
<222>  (1)..(22)
<223>  Extension Primer for HPV Type 82

<400>  72
gtgaaaccca ggtgtaataa cg                                                 22


<210>  73
<211>  20
<212>  DNA
<213>  Artificial

<220>
<223>  Artificial Sequence


<220>
<221>  misc_feature
<222>  (1)..(20)
<223>  FOrward primer for HPV Type 16

<400>  73
gtatatagag atgggaatcc                                                    20

<210>  74
<211>  20
<212>  DNA
<213>  Artificial

<220>
<223>  Artificial sequence


<220>
<221>  misc_feature
<222>  (1)..(20)
<223>  Forward Primer for HPV Type 33

<400>  74
gtatatagag agggaaatcc                                                    20


<210>  75
<211>  24
<212>  DNA
<213>  Artificial

<220>
<223>  Artificial sequence


<220>
<221>  misc_feature
<222>  (1)..(24)
<223>  Backward primer for HPV Type 16

<400>  75
aattgctcat aacagtagag atca                                               24


<210>  76
<211>  24
<212>  DNA
<213>  Artificial

<220>
<223>  Artificial Sequence


<220>
<221>  misc_feature
<222>  (1)..(24)
<223>  Backward primer for HPV Type 18

<400>  76
aattgctcgt gacatagaag gtca                                               24


<210>  77
<211>  24
<212>  DNA
<213>  Artificial

<220>
<223>  Artificial sequence

<220>
<221>  misc_feature
<222>  (1)..(24)
<223>  Backward Primer for HPV Type 26

<400>  77
aattgttcgt agcagcgtag gtca                                              24


<210>  78
<211>  24
<212>  DNA
<213>  Artificial

<220>
<223>  Artificial sequence


<220>
<221>  misc_feature
<222>  (1)..(24)
<223>  backward primer for HPV type 31

<400>  78
aattgctcat aacagtggag gtca                                              24


<210>  79
<211>  24
<212>  DNA
<213>  Artificial

<220>
<223>  Artificial sequence


<220>
<221>  misc_feature
<222>  (1)..(24)
<223>  Backward primer for HPV type 33

<400>  79
aattgctcat agcagtatag gtca                                              24


<210>  80
<211>  24
<212>  DNA
<213>  Artificial

<220>
<223>  Artificial sequence


<220>
<221>  misc_feature
<222>  (1)..(24)
<223>  Backward Primer for HPV Type 35

<400>  80
aattgctcat aacagtatag gtca                                              24

<210>  81
<211>  24
<212>  DNA
<213>  Artificial

<220>
<223>  Artificial sequence


<220>
<221>  misc_feature
<222>  (1)..(24)
<223>  Backward primer for HPV Type 39

<400>  81
aattgctcgt gacatacaag gtca                                              24


<210>  82
<211>  24
<212>  DNA
<213>  Artificial

<220>
<223>  Artificial sequence


<220>
<221>  misc_feature
<222>  (1)..(24)
<223>  Backward Primer for HPV Type 45

<400>  82
aattgctcgt aacacaacag gtca                                              24


<210>  83
<211>  24
<212>  DNA
<213>  Artificial

<220>
<223>  Artificial sequence


<220>
<221>  misc_feature
<222>  (1)..(24)
<223>  Backward primer for HPV type 51

<400>  83
aattgctcgt agcattgcaa gtca                                              24


<210>  84
<211>  24
<212>  DNA
<213>  Artificial

<220>
<223>  Artificial sequence

<220>
<221>  misc_feature
<222>  (1)..(24)
<223>  Backward primer for HPV type 52

<400>  84
aattgctcat agcagtgtag gtca                                              24


<210>  85
<211>  24
<212>  DNA
<213>  Artificial

<220>
<223>  Artificial sequence


<220>
<221>  misc_feature
<222>  (1)..(24)
<223>  Backward primer for HPV type 53

<400>  85
aattgctcat ggcattgcag gtca                                              24


<210>  86
<211>  24
<212>  DNA
<213>  Artificial

<220>
<223>  Artificial sequence


<220>
<221>  misc_feature
<222>  (1)..(24)
<223>  Backward primer for HPV type 56

<400>  86
aattgctcat tgcactgtag gtca                                              24


<210>  87
<211>  24
<212>  DNA
<213>  Artificial

<220>
<223>  Artificial sequence


<220>
<221>  misc_feature
<222>  (1)..(24)
<223>  Backward primer for HPV type 58

<400>  87
aattgctcat agcagaatag gtca                                              24

<210>  88
<211>  24
<212>  DNA
<213>  Artificial

<220>
<223>  Artificial sequence


<220>
<221>  misc_feature
<222>  (1)..(24)
<223>  Backward primer for HPV type 59

<400>  88
aattgctcgt agcacacaag gtca                                              24


<210>  89
<211>  24
<212>  DNA
<213>  Artificial

<220>
<223>  Artificial sequence


<220>
<221>  misc_feature
<222>  (1)..(24)
<223>  Backward primer for HPV type 66

<400>  89
aattgctcat tgcattgtag gtca                                              24


<210>  90
<211>  24
<212>  DNA
<213>  Artificial

<220>
<223>  Artificial sequence


<220>
<221>  misc_feature
<222>  (1)..(24)
<223>  Backward primer for HPV type 69

<400>  90
aattgttcgt aacactgtag gtca                                              24


<210>  91
<211>  24
<212>  DNA
<213>  Artificial

<220>
<223>  Artificial sequence

<220>
<221>  misc_feature
<222>  (1)..(24)
<223>  Backward primer for HPV type 82

<400>  91
aattgctcgt agcattgcaa gtca                                              24

SEQUENCE LISTING

<110> Autogenomics

<120> MULTIPLEXED HPV NUCLEIC ACID ANALYSIS WITH IMPROVED SPECIFICITY

<130> 100788.0023PCT

<150> US 60/532681
<151> 2003-12-23

<150> US 60/556737
<151> 2004-03-26

<160> 91

<170> PatentIn version 3.2

<210> 1
<211> 23
<212> DNA
<213> Artificial

<220>
<223> Artificial sequence; IUPAC ambiguity code applies for all
nucleotides other than A, C, G, and T.

<220>
<221> misc_feature
<222> (1)..(23)
<223> Forward Primer for HPV Type 6

<400> 1
gcaacaacag ttgaagaaga aac 23

<210> 2
<211> 23
<212> DNA
<213> Artificial

<220>
<223> Artificial sequence; IUPAC ambiguity code applies for all
nucleotides other than A, C, G, and T.

<220>
<221> misc_feature
<222> (1)..(23)
<223> Forward Primer for HPV Type 11

<400> 2
gcacctacag tagaagaaga aac 23

<210> 3
<211> 20
<212> DNA

<213> Artificial

<220>
<223> Artificial sequence

<220>
<221> misc_feature
<222> (1)..(20)
<223> Forward Primer for HPV Type 16

<220>
<221> misc_feature
<222> (12)..(12)
<223> K is G or T

<220>
<221> misc_feature
<222> (15)..(15)
<223> R is A or G

<400> 3
gtatatagag akggraatcc 20

<210> 4
<211> 21
<212> DNA
<213> Artificial

<220>
<223> Artificial sequence

<220>
<221> misc_feature
<222> (1)..(21)
<223> Forward Primer for HPV Type 18

<400> 4
gtgtatagag acagtatacc g 21

<210> 5
<211> 20
<212> DNA
<213> Artificial

<220>
<223> Artificial sequence

<220>
<221> misc_feature
<222> (1)..(20)
<223> Forward Primer for HPV Type 26

<400> 5
gtatatagag ataggagtcc 20

<210> 6
<211> 20
<212> DNA
<213> Artificial

<220>
<223> Artificial sequence


<220>
<221> misc_feature
<222> (1)..(20)
<223> Forward Primer for HPV Type 31

<400> 6
gtatataggg acgacacacc                                20


<210> 7
<211> 20
<212> DNA
<213> Artificial

<220>
<223> Artificial sequence


<220>
<221> misc_feature
<222> (1)..(20)
<223> Forward Primer for HPV Type 33

<220>
<221> misc_feature
<222> (12)..(12)
<223> K is G or T

<220>
<221> misc_feature
<222> (15)..(15)
<223> R is A or G

<400> 7
gtatatagag akggraatcc                                20


<210> 8
<211> 20
<212> DNA
<213> Artificial

<220>
<223> Artificial sequence


<220>
<221> misc_feature

<222> (1)..(20)
<223> Forward Primer for HPV Type 35

<400> 8
gtatatagag aaggccagcc 20

<210> 9
<211> 20
<212> DNA
<213> Artificial

<220>
<223> Artificial sequence

<220>
<221> misc_feature
<222> (1)..(20)
<223> Forward Primer for HPV Type 39

<400> 9
gtatataggg acggggaacc 20

<210> 10
<211> 23
<212> DNA
<213> Artificial

<220>
<223> Artificial sequence

<220>
<221> misc_feature
<222> (1)..(23)
<223> Forward Primer for HPV Type 42

<400> 10
tggtatacag tggagaaaga aac 23

<210> 11
<211> 23
<212> DNA
<213> Artificial

<220>
<223> Artificial sequence

<220>
<221> misc_feature
<222> (1)..(23)
<223> Forward Primer for HPV Type 43

<400> 11
gcagatactg tagaagaaga aac 23

<210> 12
<211> 23
<212> DNA
<213> Artificial

<220>
<223> Artificial sequence

<220>
<221> misc_feature
<222> (1)..(23)
<223> Forward Primer for HPV Type 44

<400> 12
gcagtaacag tggaagaaga aac 23

<210> 13
<211> 20
<212> DNA
<213> Artificial

<220>
<223> Artificial sequence

<220>
<221> misc_feature
<222> (1)..(20)
<223> Forward Primer for HPV Type 45

<400> 13
gtgtatagag actgtatagc 20

<210> 14
<211> 26
<212> DNA
<213> Artificial

<220>
<223> Artificial sequence

<220>
<221> misc_feature
<222> (1)..(26)
<223> Forward Primer for HPV Type 51

<400> 14
gattgtatat agggataata atccat 26

<210> 15
<211> 25
<212> DNA

<213> Artificial

<220>
<223> Artificial sequence

<220>
<221> misc_feature
<222> (1)..(25)
<223> Forward Primer for HPV Type 52

<400> 15
gaatagtata tagagacaat aatcc 25

<210> 16
<211> 20
<212> DNA
<213> Artificial

<220>
<223> Artificial sequence

<220>
<221> misc_feature
<222> (1)..(20)
<223> Forward Primer for HPV Type 53

<400> 16
gtgtatagag acgggtatcc 20

<210> 17
<211> 20
<212> DNA
<213> Artificial

<220>
<223> Artificial sequence

<220>
<221> misc_feature
<222> (1)..(20)
<223> Forward Primer for HPV Type 56

<400> 17
gtgtataggg atgattttcc 20

<210> 18
<211> 20
<212> DNA
<213> Artificial

<220>
<223> Artificial sequence

<220>
<221> misc_feature
<222> (1)..(20)
<223> Forward Primer for HPV Type 58

<400> 18
gtgtatagag atggaaatcc                                    20


<210> 19
<211> 20
<212> DNA
<213> Artificial

<220>
<223> Artificial sequence


<220>
<221> misc_feature
<222> (1)..(20)
<223> Forward Primer for HPV Type 59

<400> 19
gtgtatagag actgtagacc                                    20


<210> 20
<211> 24
<212> DNA
<213> Artificial

<220>
<223> Artificial sequence


<220>
<221> misc_feature
<222> (1)..(24)
<223> Forward Primer for HPV Type 66

<400> 20
aactagtata tagaaacaat tggc                               24


<210> 21
<211> 21
<212> DNA
<213> Artificial

<220>
<223> Artificial sequence


<220>
<221> misc_feature
<222> (1)..(21)
<223> Forward Primer for HPV Type 68

<400> 21
gtagtatata gggacggggt a                                    21

<210> 22
<211> 25
<212> DNA
<213> Artificial

<220>
<223> Artificial sequence

<220>
<221> misc_feature
<222> (1)..(25)
<223> Forward Primer for HPV Type 69

<400> 22
gaatagtgta tagaaatgat agtgc                                25

<210> 23
<211> 25
<212> DNA
<213> Artificial

<220>
<223> Artificial sequence

<220>
<221> misc_feature
<222> (1)..(25)
<223> Forward Primer for HPV Type 73

<400> 23
gtattgtata tagaaaggat aaacc                                25

<210> 24
<211> 19
<212> DNA
<213> Artificial

<220>
<223> Artificial sequence

<220>
<221> misc_feature
<222> (1)..(19)
<223> Forward Primer for HPV Type 82

<400> 24
gtatatagga caatacgcc                                       19

<210> 25
<211> 23
<212> DNA
<213> Artificial

<220>
<223> Artificial sequence


<220>
<221> misc_feature
<222> (1)..(23)
<223> Backward Primer for HPV Type 6

<400> 25
agctgttgca cttctctgat gtc                                23


<210> 26
<211> 23
<212> DNA
<213> Artificial

<220>
<223> Artificial sequence


<220>
<221> misc_feature
<222> (1)..(23)
<223> Backward Primer for HPV Type 11

<400> 26
aggtcttgta gttgtctgat gtc                                23


<210> 27
<211> 24
<212> DNA
<213> Artificial

<220>
<223> Artificial sequence


<220>
<221> misc_feature
<222> (1)..(24)
<223> Backward Primer for HPV Type 16

<220>
<221> misc_feature
<222> (17)..(17)
<223> R is A or G

<220>
<221> misc_feature
<222> (21)..(21)
<223> R is A or G

<400> 27
aattgctcat aacagtrgag rtca                                24

<210> 28
<211> 24
<212> DNA
<213> Artificial

<220>
<223> Artificial sequence

<220>
<221> misc_feature
<222> (1)..(24)
<223> Backward Primer for HPV Type 18

<220>
<221> misc_feature
<222> (17)..(17)
<223> S is C or G

<400> 28
aattgctcgt gacatasaag gtca                                24

<210> 29
<211> 24
<212> DNA
<213> Artificial

<220>
<223> Artificial sequence

<220>
<221> misc_feature
<222> (1)..(24)
<223> Backward Primer for HPV Type 26

<220>
<221> misc_feature
<222> (12)..(12)
<223> R is A or G

<220>
<221> misc_feature
<222> (15)..(15)
<223> S is C or G

<220>
<221> misc_feature
<222> (16)..(16)
<223> Y is C or T

<400> 29
aattgttcgt arcasygtag gtca                                24

<210> 30
<211> 24
<212> DNA
<213> Artificial

<220>
<223> Artificial sequence


<220>
<221> misc_feature
<222> (1)..(24)
<223> Backward Primer for HPV Type 31

<220>
<221> misc_feature
<222> (17)..(17)
<223> R is A or G

<220>
<221> misc_feature
<222> (21)..(21)
<223> R is A or G

<400> 30
aattgctcat aacagtrgag rtca                    24


<210> 31
<211> 24
<212> DNA
<213> Artificial

<220>
<223> Artificial sequence


<220>
<221> misc_feature
<222> (1)..(24)
<223> Backward Primer for HPV Type 33

<220>
<221> misc_feature
<222> (12)..(12)
<223> R is A or G

<400> 31
aattgctcat arcagtatag gtca                    24


<210> 32
<211> 24
<212> DNA
<213> Artificial

<220>

<223> Artificial sequence

<220>
<221> misc_feature
<222> (1)..(24)
<223> Backward Primer for HPV Type 35

<220>
<221> misc_feature
<222> (12)..(12)
<223> R is A or G

<400> 32
aattgctcat arcagtatag gtca 24

<210> 33
<211> 24
<212> DNA
<213> Artificial

<220>
<223> Artificial sequence

<220>
<221> misc_feature
<222> (1)..(24)
<223> Backward Primer for HPV Type 39

<220>
<221> misc_feature
<222> (17)..(17)
<223> S is C or G

<400> 33
aattgctcgt gacatasaag gtca 24

<210> 34
<211> 20
<212> DNA
<213> Artificial

<220>
<223> Artificial sequence

<220>
<221> misc_feature
<222> (1)..(20)
<223> Backward Primer for HPV Type 42

<400> 34
cccaaaagca tctgttgcag 20

<210> 35

<211> 23
<212> DNA
<213> Artificial

<220>
<223> Artificial sequence


<220>
<221> misc_feature
<222> (1)..(23)
<223> Backward Primer for HPV Type 43

<400> 35
cagtcttcta gcttcttgat gtc                                    23


<210> 36
<211> 23
<212> DNA
<213> Artificial

<220>
<223> Artificial sequence


<220>
<221> misc_feature
<222> (1)..(23)
<223> Backward Primer for HPV Type 44

<400> 36
agcgtatgta ggtgatggat gtc                                    23


<210> 37
<211> 24
<212> DNA
<213> Artificial

<220>
<223> Artificial sequence


<220>
<221> misc_feature
<222> (1)..(24)
<223> Backward Primer for HPV Type 45

<220>
<221> misc_feature
<222> (12)..(12)
<223> R is A or G

<220>
<221> misc_feature
<222> (17)..(18)
<223> M is A or C

<400> 37
aattgctcgt arcacammag gtca                                24

<210> 38
<211> 24
<212> DNA
<213> Artificial

<220>
<223> Artificial sequence

<220>
<221> misc_feature
<222> (1)..(24)
<223> Backward Primer for HPV Type 51

<220>
<221> misc_feature
<222> (9)..(9)
<223> R is A or G

<220>
<221> misc_feature
<222> (11)..(11)
<223> R is A or G

<220>
<221> misc_feature
<222> (20)..(20)
<223> R is A or G

<400> 38
aattgctcrt rgcattgcar gtca                                24

<210> 39
<211> 24
<212> DNA
<213> Artificial

<220>
<223> Artificial sequence

<220>
<221> misc_feature
<222> (1)..(24)
<223> Backward Primer for HPV Type 52

<220>
<221> misc_feature
<222> (16)..(16)
<223> W is A or T

<400> 39
aattgctcat agcagwrtag gtca                                24

<210> 40
<211> 24
<212> DNA
<213> Artificial

<220>
<223> Artificial sequence


<220>
<221> misc_feature
<222> (1)..(24)
<223> Backward Primer for HPV Type 53

<220>
<221> misc_feature
<222> (9)..(9)
<223> R is A or G

<220>
<221> misc_feature
<222> (11)..(11)
<223> R is A or G

<220>
<221> misc_feature
<222> (20)..(20)
<223> R is A or G

<400> 40
aattgctcrt rgcattgcar gtca                                    24


<210> 41
<211> 24
<212> DNA
<213> Artificial

<220>
<223> Artificial sequence


<220>
<221> misc_feature
<222> (1)..(24)
<223> Backward Primer for HPV Type 56

<220>
<221> misc_feature
<222> (15)..(15)
<223> Y is C or T

<400> 41
aattgctcat tgcaytgtag gtca                                    24


<210> 42
<211> 24

<212> DNA
<213> Artificial

<220>
<223> Artificial sequence


<220>
<221> misc_feature
<222> (1)..(24)
<223> Backward Primer for HPV Type 58

<220>
<221> misc_feature
<222> (16)..(16)
<223> W is A or T

<220>
<221> misc_feature
<222> (17)..(17)
<223> R is A or G

<400> 42
aattgctcat agcagwrtag gtca                                24


<210> 43
<211> 24
<212> DNA
<213> Artificial

<220>
<223> Artificial sequence


<220>
<221> misc_feature
<222> (1)..(24)
<223> Backward Primer for HPV Type 59

<220>
<221> misc_feature
<222> (12)..(12)
<223> R is A or G

<220>
<221> misc_feature
<222> (17)..(18)
<223> M is A or C

<400> 43
aattgctcgt arcacammag gtca                                24


<210> 44
<211> 24
<212> DNA
<213> Artificial

<220>
<223> Artificial sequence

<220>
<221> misc_feature
<222> (1)..(24)
<223> Backward Primer for HPV Type 66

<220>
<221> misc_feature
<222> (15)..(15)
<223> Y is C or T

<400> 44
aattgctcat tgcaytgtag gtca 24

<210> 45
<211> 24
<212> DNA
<213> Artificial

<220>
<223> Artificial sequence

<220>
<221> misc_feature
<222> (1)..(24)
<223> Backward Primer for HPV Type 68

<400> 45
aattgctcgt gacatacaag gtcg 24

<210> 46
<211> 24
<212> DNA
<213> Artificial

<220>
<223> Artificial sequence

<220>
<221> misc_feature
<222> (1)..(24)
<223> Backward Primer for HPV Type 69

<220>
<221> misc_feature
<222> (12)..(12)
<223> R is A or G

<220>
<221> misc_feature
<222> (15)..(15)
<223> S is C or G

<220>
<221> misc_feature
<222> (16)..(16)
<223> Y is C or T

<400> 46
aattgttcgt arcasygtag gtca                                    24


<210> 47
<211> 24
<212> DNA
<213> Artificial

<220>
<223> Artificial sequence


<220>
<221> misc_feature
<222> (1)..(24)
<223> Backward Primer for HPV Type 73

<400> 47
caatgactcg taacatgtaa ggtc                                    24


<210> 48
<211> 24
<212> DNA
<213> Artificial

<220>
<223> Artificial sequence


<220>
<221> misc_feature
<222> (1)..(24)
<223> Backward Primer for HPV Type 82

<220>
<221> misc_feature
<222> (9)..(9)
<223> R is A or G

<220>
<221> misc_feature
<222> (11)..(11)
<223> R is A or G

<220>
<221> misc_feature
<222> (20)..(20)
<223> R is A or G

<400> 48
aattgctcrt rgcattgcar gtca                                    24

<210> 49
<211> 20
<212> DNA
<213> Artificial

<220>
<223> Artificial sequence


<220>
<221> misc_feature
<222> (1)..(20)
<223> Extension Primer for HPV Type 6

<400> 49
gaagtggacg gacaagattc                                20


<210> 50
<211> 20
<212> DNA
<213> Artificial

<220>
<223> Artificial sequence


<220>
<221> misc_feature
<222> (1)..(20)
<223> Extension Primer for HPV Type 11

<400> 50
caaggtggac aacacagacg                                20


<210> 51
<211> 22
<212> DNA
<213> Artificial

<220>
<223> Artificial sequence


<220>
<221> misc_feature
<222> (1)..(22)
<223> Extension Primer for HPV Type 16

<400> 51
gcatggagat acacctacat tg                             22


<210> 52
<211> 21
<212> DNA

<213> Artificial

<220>
<223> Artificial sequence

<220>
<221> misc_feature
<222> (1)..(21)
<223> Extension Primer for HPV Type 18

<400> 52
gacaggaacg actccaacga c 21

<210> 53
<211> 20
<212> DNA
<213> Artificial

<220>
<223> Artificial sequence

<220>
<221> misc_feature
<222> (1)..(20)
<223> Extension Primer for HPV Type 26

<400> 53
gagaccaagg cgccaaacag 20

<210> 54
<211> 22
<212> DNA
<213> Artificial

<220>
<223> Artificial sequence

<220>
<221> misc_feature
<222> (1)..(22)
<223> Extension Primer for HPV Type 31

<400> 54
catgcgtgga gaaacaccta cg 22

<210> 55
<211> 21
<212> DNA
<213> Artificial

<220>
<223> Artificial sequence

<220>
<221> misc_feature
<222> (1)..(21)
<223> Extension Primer for HPV Type 33

<400> 55
cgacgtagag aaactgcact g                                21


<210> 56
<211> 20
<212> DNA
<213> Artificial

<220>
<223> Artificial sequence


<220>
<221> misc_feature
<222> (1)..(20)
<223> Extension Primer for HPV Type 35

<400> 56
gacaggtcgg tgtatgtcct                                20


<210> 57
<211> 21
<212> DNA
<213> Artificial

<220>
<223> Artificial sequence


<220>
<221> misc_feature
<222> (1)..(21)
<223> Extension Primer for HPV Type 39

<400> 57
gaccgcagac taacacgaag a                                21


<210> 58
<211> 20
<212> DNA
<213> Artificial

<220>
<223> Artificial sequence


<220>
<221> misc_feature
<222> (1)..(20)
<223> Extension Primer for HPV Type 42

<400> 58
tgaccaagcc aaacaggaca                                20

<210> 59
<211> 20
<212> DNA
<213> Artificial

<220>
<223> Artificial sequence

<220>
<221> misc_feature
<222> (1)..(20)
<223> Extension Primer for HPV Type 43

<400> 59
ggaccagcaa gtgaatctac                                20

<210> 60
<211> 20
<212> DNA
<213> Artificial

<220>
<223> Artificial sequence

<220>
<221> misc_feature
<222> (1)..(20)
<223> Extension Primer for HPV Type 44

<400> 60
cgcaagacgt tacacagcct                                20

<210> 61
<211> 21
<212> DNA
<213> Artificial

<220>
<223> Artificial sequence

<220>
<221> misc_feature
<222> (1)..(21)
<223> Extension Primer for HPV Type 45

<400> 61
gaaagacttc gcagacgtag g                              21

<210> 62
<211> 20
<212> DNA
<213> Artificial

<220>
<223> Artificial sequence

<220>
<221> misc_feature
<222> (1)..(20)
<223> Extension Primer for HPV Type 51

<400> 62
acgtacacga caacgtaacg 20

<210> 63
<211> 21
<212> DNA
<213> Artificial

<220>
<223> Artificial sequence

<220>
<221> misc_feature
<222> (1)..(21)
<223> Extension Primer for HPV Type 52

<400> 63
ctgtgaccca agtgtaacgt c 21

<210> 64
<211> 18
<212> DNA
<213> Artificial

<220>
<223> Artificial sequence

<220>
<221> misc_feature
<222> (1)..(18)
<223> Extension Primer for HPV Type 53

<400> 64
gaccgggtcg tgcctgac 18

<210> 65
<211> 21
<212> DNA
<213> Artificial

<220>
<223> Artificial sequence

<220>
<221> misc_feature
<222> (1)..(21)
<223> Extension Primer for HPV Type 56

<400> 65
gagacaaaca tctagagaac c                                    21

<210> 66
<211> 19
<212> DNA
<213> Artificial

<220>
<223> Artificial sequence

<220>
<221> misc_feature
<222> (1)..(19)
<223> Extension Primer for HPV Type 58

<400> 66
gacagggcgc tgtgcagtg                                    19

<210> 67
<211> 19
<212> DNA
<213> Artificial

<220>
<223> Artificial sequence

<220>
<221> misc_feature
<222> (1)..(19)
<223> Extension Primer for HPV Type 59

<400> 67
caaagacaag cgcgtagtg                                    19

<210> 68
<211> 24
<212> DNA
<213> Artificial

<220>
<223> Artificial sequence

<220>

<221> misc_feature
<222> (1)..(24)
<223> Extension Primer for HPV Type 66

<400> 68
gacatacgag tagacaagct acag 24

<210> 69
<211> 18
<212> DNA
<213> Artificial

<220>
<223> Artificial sequence

<220>
<221> misc_feature
<222> (1)..(18)
<223> Extension Primer for HPV Type 68

<400> 69
gcagacgcac acggcagg 18

<210> 70
<211> 20
<212> DNA
<213> Artificial

<220>
<223> Artificial sequence

<220>
<221> misc_feature
<222> (1)..(20)
<223> Extension Primer for HPV Type 69

<400> 70
ggatgaaaag cgacggttcc 20

<210> 71
<211> 23
<212> DNA
<213> Artificial

<220>
<223> Artificial sequence

<220>
<221> misc_feature
<222> (1)..(23)
<223> Extension Primer for HPV Type 73

<400> 71

cggtttcatc aaatagcaga aca                    23

<210> 72
<211> 22
<212> DNA
<213> Artificial

<220>
<223> Artificial sequence

<220>
<221> misc_feature
<222> (1)..(22)
<223> Extension Primer for HPV Type 82

<400> 72
gtgaaaccca ggtgtaataa cg                    22

<210> 73
<211> 20
<212> DNA
<213> Artificial

<220>
<223> Artificial Sequence

<220>
<221> misc_feature
<222> (1)..(20)
<223> Forward primer for HPV Type 16

<400> 73
gtatatagag atgggaatcc                    20

<210> 74
<211> 20
<212> DNA
<213> Artificial

<220>
<223> Artificial sequence

<220>
<221> misc_feature
<222> (1)..(20)
<223> Forward Primer for HPV Type 33

<400> 74
gtatatagag agggaaatcc                    20

<210> 75
<211> 24

<212> DNA
<213> Artificial

<220>
<223> Artificial sequence


<220>
<221> misc_feature
<222> (1)..(24)
<223> Backward primer for HPV Type 16

<400> 75
aattgctcat aacagtagag atca                                24


<210> 76
<211> 24
<212> DNA
<213> Artificial

<220>
<223> Artificial Sequence


<220>
<221> misc_feature
<222> (1)..(24)
<223> Backward primer for HPV Type 18

<400> 76
aattgctcgt gacatagaag gtca                                24


<210> 77
<211> 24
<212> DNA
<213> Artificial

<220>
<223> Artificial sequence


<220>
<221> misc_feature
<222> (1)..(24)
<223> Backward Primer for HPV Type 26

<400> 77
aattgttcgt agcagcgtag gtca                                24


<210> 78
<211> 24
<212> DNA
<213> Artificial

<220>
<223> Artificial sequence

<220>
<221> misc_feature
<222> (1)..(24)
<223> backward primer for HPV type 31

<400> 78
aattgctcat aacagtggag gtca 24

<210> 79
<211> 24
<212> DNA
<213> Artificial

<220>
<223> Artificial sequence

<220>
<221> misc_feature
<222> (1)..(24)
<223> Backward primer for HPV type 33

<400> 79
aattgctcat agcagtatag gtca 24

<210> 80
<211> 24
<212> DNA
<213> Artificial

<220>
<223> Artificial sequence

<220>
<221> misc_feature
<222> (1)..(24)
<223> Backward Primer for HPV Type 35

<400> 80
aattgctcat aacagtatag gtca 24

<210> 81
<211> 24
<212> DNA
<213> Artificial

<220>
<223> Artificial sequence

<220>
<221> misc_feature
<222> (1)..(24)

<223> Backward primer for HPV Type 39

<400> 81
aattgctcgt gacatacaag gtca 24

<210> 82
<211> 24
<212> DNA
<213> Artificial

<220>
<223> Artificial sequence

<220>
<221> misc_feature
<222> (1)..(24)
<223> Backward Primer for HPV Type 45

<400> 82
aattgctcgt aacacaacag gtca 24

<210> 83
<211> 24
<212> DNA
<213> Artificial

<220>
<223> Artificial sequence

<220>
<221> misc_feature
<222> (1)..(24)
<223> Backward primer for HPV type 51

<400> 83
aattgctcgt agcattgcaa gtca 24

<210> 84
<211> 24
<212> DNA
<213> Artificial

<220>
<223> Artificial sequence

<220>
<221> misc_feature
<222> (1)..(24)
<223> Backward primer for HPV type 52

<400> 84
aattgctcat agcagtgtag gtca 24

<210> 85
<211> 24
<212> DNA
<213> Artificial

<220>
<223> Artificial sequence


<220>
<221> misc_feature
<222> (1)..(24)
<223> Backward primer for HPV type 53

<400> 85
aattgctcat ggcattgcag gtca 24


<210> 86
<211> 24
<212> DNA
<213> Artificial

<220>
<223> Artificial sequence


<220>
<221> misc_feature
<222> (1)..(24)
<223> Backward primer for HPV type 56

<400> 86
aattgctcat tgcactgtag gtca 24


<210> 87
<211> 24
<212> DNA
<213> Artificial

<220>
<223> Artificial sequence


<220>
<221> misc_feature
<222> (1)..(24)
<223> Backward primer for HPV type 58

<400> 87
aattgctcat agcagaatag gtca 24


<210> 88
<211> 24
<212> DNA
<213> Artificial

<220>
<223> Artificial sequence

<220>
<221> misc_feature
<222> (1)..(24)
<223> Backward primer for HPV type 59

<400> 88
aattgctcgt agcacacaag gtca 24

<210> 89
<211> 24
<212> DNA
<213> Artificial

<220>
<223> Artificial sequence

<220>
<221> misc_feature
<222> (1)..(24)
<223> Backward primer for HPV type 66

<400> 89
aattgctcat tgcattgtag gtca 24

<210> 90
<211> 24
<212> DNA
<213> Artificial

<220>
<223> Artificial sequence

<220>
<221> misc_feature
<222> (1)..(24)
<223> Backward primer for HPV type 69

<400> 90
aattgttcgt aacactgtag gtca 24

<210> 91
<211> 24
<212> DNA
<213> Artificial

<220>
<223> Artificial sequence

<220>
<221> misc_feature
<222> (1)..(24)
<223> Backward primer for HPV type 82

<400> 91

aattgctcgt agcattgcaa gtca 24

## Claims

1. A synthetic nucleic acid that has less than sixty nucleotides and comprising an HPV recognition sequence selected from the group consisting of SEQ ID Ax, SEQ ID Bx, and SEQ ID Cx, wherein X is an integer between 1 and 24, wherein no more than two nucleotides in the HPV recognition sequence are replaced by N.

2. The synthetic nucleic acid of claim 1 having SEQ ID Cx and further comprising a plurality of nucleotides at the 5'-terminus that have less than 60% homology to a target sequence to which the nucleic acid hybridizes.

3. The synthetic nucleic acid of claim 1 wherein SEQ ID Ax, SEQ ID Bx, and SEQ ID Cx are complementary to an HPV mutant.

4. The synthetic nucleic acid of claim 1 having less than 40 nucleotides.

5. The synthetic nucleic acid of claim 1 consisting of the HPV recognition sequence.
